# EUROPEAN PATENT SPECIFICATION

(11) **EP 4 299 106 B1**
(45) Date of publication and mention of the grant of the patent: **03.09.2025**
(21) Application number: 23203890.1
(22) Date of filing: 24.01.2018
(51) Int. Cl.: A61B 5/00, A61B 5/296, A61N 1/00, A61N 1/05, A61N 1/36, A61N 1/372, A61B 5/08, A61N 1/04, A61N 2/00, A61N 2/02

(54) **ACCESSING SPINAL NETWORK TO ENABLE RESPIRATORY FUNCTION**
ZUGRIFF AUF EIN SPINALES NETZWERK ZUR ERMÖGLICHUNG DER ATEMFUNKTION
ACCÈS À UN RÉSEAU VERTÉBRAL POUR PERMETTRE LA FONCTION RESPIRATOIRE

(30) Priority: 24.01.2017 US 201762449993 P
(43) Date of publication of application: 03.01.2024
(62) Divisional of application: 18744685.1
(73) Proprietor: The Regents of The University of California, Oakland CA 94607-5200 (US)
(72) Inventor: LU, Daniel C., Los Angeles, 940049 (US)
(74) Representative: Mewburn Ellis LLP

(56) References cited:
- WO-A1-2017/011410
- US-A1- 2015 231 396

## Description

### BACKGROUND

Trauma, disease, infection, drugs, and other factors can diminish the human body's ability to breathe and in such instances, it is difficult to mechanically mimic the human body's method of respiration.

Respiration or breathing involves a complex network of circuits that is involved in central pattern generation (CPG) that spans the brainstem and cervical spinal cord to generate a respiratory rhythm. Sensory input from central and peripheral chemoreceptors (*e.g.,* CO₂ receptors), lung stretch receptors naturally influence the firing pattern of the CPG. In the disease or injury state to the brainstem or spinal cord, the respiratory rhythm is compromised, likely due to the depressed state of the CPG. Current technology in addressing the depressed respiratory state is to stimulate the diaphragm muscle which actively participates in inspiration by phrenic nerve stimulators. The issue with this approach is that the muscle will not react to changes in the activity state of the patient and only activates the diaphragm which participates in the inspiratory phase of respiration. WO 2017/011410 Al discloses a method for improving, regulating and restoring respiration, involves neuromodulating cervical spinal cord of subject with magnetic stimulator at frequency and intensity sufficient to regulate and to restore respiration.

US 2015/231396 Al discloses a neurostimulator system for patients having neural conditions and symptoms e.g., epilepsy, has processor that modifies complex stimulation pattern delivered by stimulation assembly based on signals received from sensor interface.

### DEFINITIONS

As used herein "electrical stimulation" or "stimulation" means application of an electrical signal that may be either excitatory or inhibitory to a muscle or neuron and/or to groups of neurons and/or interneurons. It will be understood that an electrical signal may be applied to one or more electrodes with one or more return electrodes.

As used herein "magnetic stimulation" or means use of a varying magnetic field to induce an electrical signal, *e.g.,* in a neuron, that may be either excitatory or inhibitory to a muscle or neuron and/or to groups of neurons and/or interneurons. It will be understood that an electrical signal may be applied to one or more electrodes with one or more return electrodes.

As used herein "epidural" means situated upon the dura or in very close proximity to the dura. The term "epidural stimulation" refers to electrical epidural stimulation.

The term "transcutaneous stimulation" or "transcutaneous electrical stimulation" or "cutaneous electrical stimulation" refers to electrical stimulation applied to the skin, and, as typically used herein refers to electrical stimulation applied to the skin in order to effect stimulation of the spinal cord or a region thereof. The term "transcutaneous electrical spinal cord stimulation" may also be referred to as "tSCS". The term "pcEmc" refers to painless cutaneous electrical stimulation.

The term "motor complete" when used with respect to a spinal cord injury indicates that there is no motor function below the lesion, (*e.g.,* no movement can be voluntarily induced in muscles innervated by spinal segments below the spinal lesion.

The term "monopolar stimulation" refers to stimulation between a local electrode and a common distant return electrode.

The term "co-administering", "concurrent administration", "administering in conjunction with" or "administering in combination" when used, for example with respect to transcutaneous electrical stimulation, epidural electrical stimulation, and pharmaceutical administration, refers to administration of the transcutaneous electrical stimulation and/or epidural electrical stimulation and/or pharmaceutical such that various modalities can simultaneously achieve a physiological effect on the subject. The administered modalities need not be administered together, either temporally or at the same site. In some embodiments, the various "treatment" modalities are administered at different times. In some embodiments, administration of one can precede administration of the other (*e.g.,* drug before electrical and/or magnetic stimulation or vice versa). Simultaneous physiological effect need not necessarily require presence of drug and the electrical and/or magnetic stimulation at the same time or the presence of both stimulation modalities at the same time. In some embodiments, all the modalities are administered essentially simultaneously.

The phrase "spinal cord stimulation" as used herein includes stimulation of any spinal nervous tissue, including spinal neurons, accessory neuronal cells, nerves, nerve roots, nerve fibers, or tissues, that are associated with the spinal cord. It is contemplated that spinal cord stimulation may comprise stimulation of one or more areas associated with a cervical vertebral segment.

As used herein, "spinal nervous tissue" refers to nerves, neurons, neuroglial cells, glial cells, neuronal accessory cells, nerve roots, nerve fibers, nerve rootlets, parts of nerves, nerve bundles, mixed nerves, sensory fibers, motor fibers, dorsal root, ventral root, dorsal root ganglion, spinal ganglion, ventral motor root, general somatic afferent fibers, general visceral afferent fibers, general somatic efferent fibers, general visceral efferent fibers, grey matter, white matter, the dorsal column, the lateral column, and/or the ventral column associated with the spinal cord. Spinal nervous tissue includes "spinal nerve roots," that comprise any one or more of the 31 pairs of nerves that emerge from the spinal cord. Spinal nerve roots may be cervical nerve roots, thoracic nerve roots, and lumbar nerve roots.

### SUMMARY

The invention is defined in claim 1. Further aspects and preferred embodiments are defined in the appended claims.

### BRIEF DESCRIPTION OF THE DRAWINGS

Figure 1 shows a schematic illustration of one illustrative embodiment of a magnetic nerve stimulator.
Figure 2 schematically illustrates one embodiment of a respiratory stimulation/maintenance system as described herein.
Figure 3 shows an anterolateral view of the spinal cord illustrating sites of epidural stimulation.
Figures 4A-4C illustrate epidural electrodes for stimulating respiration. Fig. 4A: Dorsal respiration electrode type A (32 channel). Fig. 4B: Dorsal respiration electrode type B (48 channel). Fig. 4C: Ventral respiration dual electrode type C (8 channel). *Inferolateral exiting electrode tail allows for insertion of electrode through posterior laminotomy approach. While illustrative dimensions are shown, it will be recognized that the dimensions can be adjusted and personalized to the patient based on anatomical dimensions based on MRI.
Figure 5, panels A-C illustrates the experimental set-up. Panel A: The experimental set-up used in this study is shown. Panel B: The epidural stimulation sites on dorsal lateral spinal cord. Panel C: The recording time line for each cervical level, with sham experiment recorded first followed by stimulation.

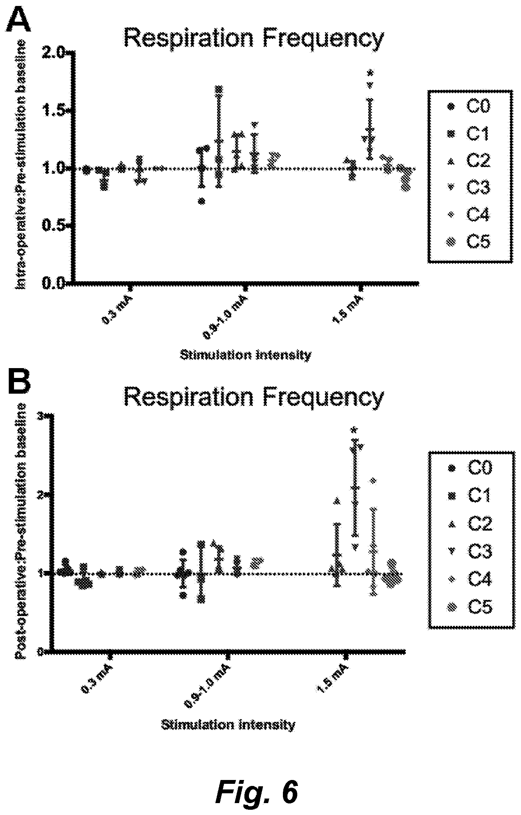

Figure 6, panels A-B, shows the determination of the current intensity to use for each cervical level tested. The electrical stimulation intensities used were 0.3, 0.9-1.0 or 1.5 mA. Panel A: The respiratory frequency ratio of intra-operative recording compared to the pre-stimulation baseline is shown, and (Panel B) the respiration frequency ratio of post-stimulation recording compared to the pre-stimulation baseline is shown. In both analyses, the stimulation intensity of 1.5 mA at C3 caused the only significant increase in the respiratory ratio (see *). For C0 and C1, the stimulation intensity of 1.5mA was too strong and induced cardiac arrest. For C5, only 1.5 mA was tested as the location was close to a major blood vessel that impeded the recording.

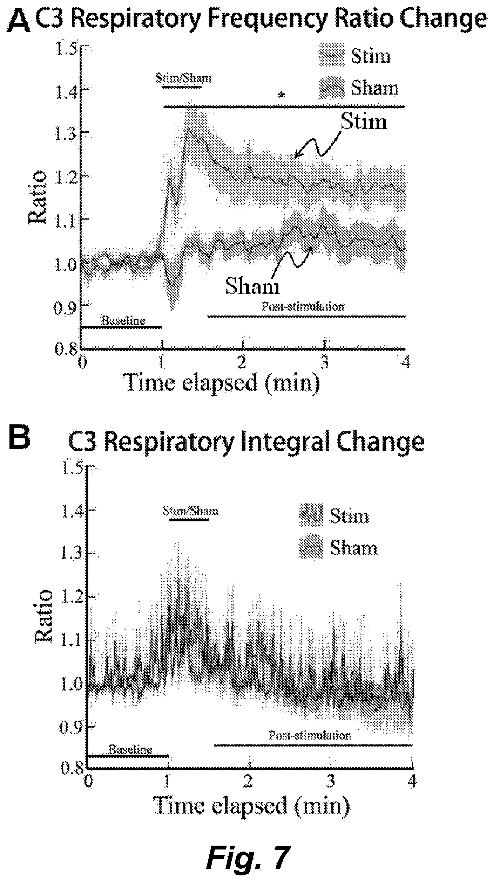

Figure 7, panels A-B, shows that Respiratory frequency increased when C3 was stimulated (20 Hz, 1.5 mA). Panel A shows the respiratory frequency change when C3 was electrically stimulated or a sham stimulation was carried out. Panel B shows the respiratory peak-to-peak amplitude ratio change when C3 was electrically stimulated or a sham stimulation was carried out. The ratio was calculated using each recorded data point compared to the calculated baseline median. (n=24). The respiratory frequency was significantly increased during epidural stimulation compared to sham stimulation (p<0.05). However, there was no change in respiratory peak to peak amplitude during epidural stimulation compared to the sham stimulation.

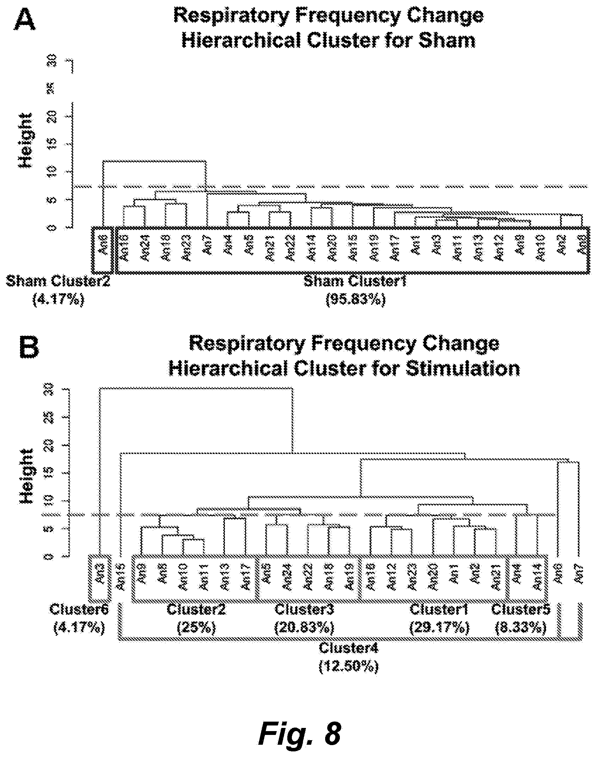

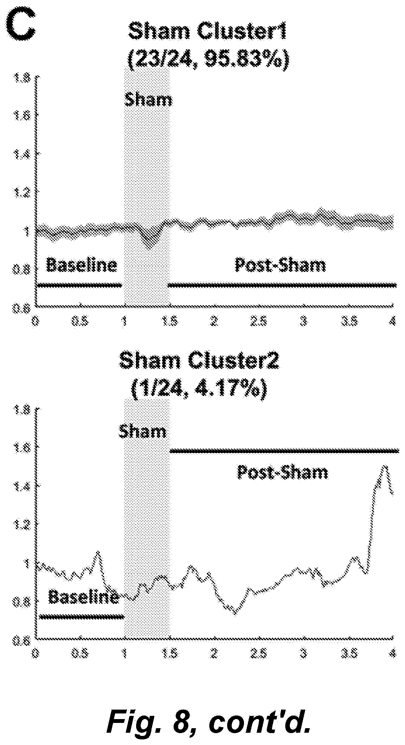

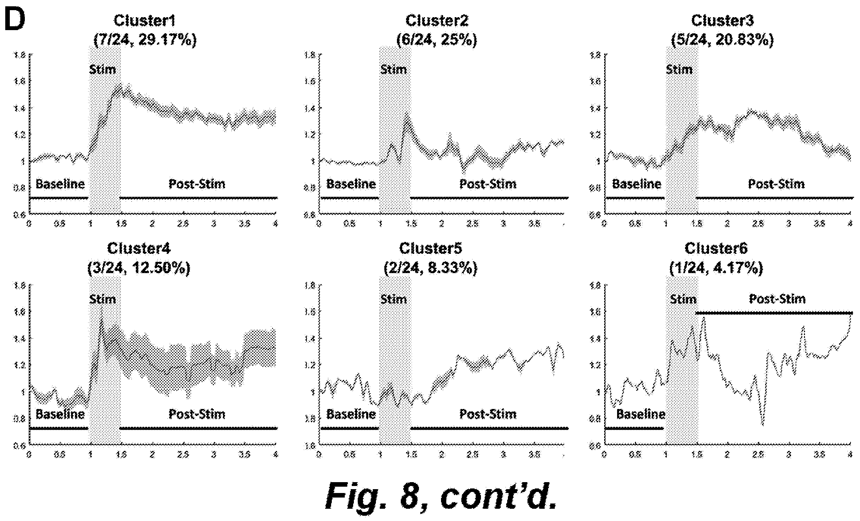

Figure 8, panels A-D, illustrates a hierarchical cluster showing different respiratory frequency change pattern due to epidural stimulation at C3 with 1.5 mA. The hierarchical cluster dendograms of the sham group (Panel A) and the epidural stimulation group (panel B) are shown above. Individual recordings were placed in separate clusters if the distance between each tracing was greater than 7.5 hierarchical units (An = animal). Panel C: Tracings of the average clusters for the sham group: (i) sham cluster 1, which consisted of data from 23 of 24 animals, and (ii) sham cluster 2 which contained data from only 1 animal. Panel D: Tracings of the average clusters for the epidural stimulation group: (i) cluster 1 contained 7 of 24 animals, (ii) cluster 2 contained 6 of 24 animals, (iii) cluster 3 contained 5 of 24 animals, (iv) cluster 4 contained 3 of 24 animals, (v) cluster 5 contained 2 of 24 animals and (vi) cluster 6 contained only 1 animal.

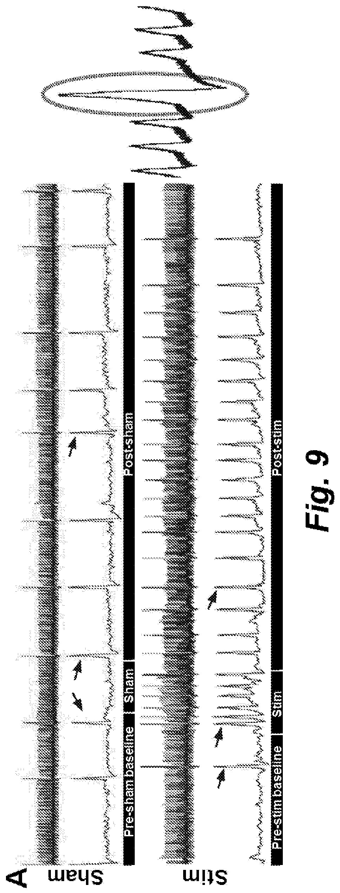

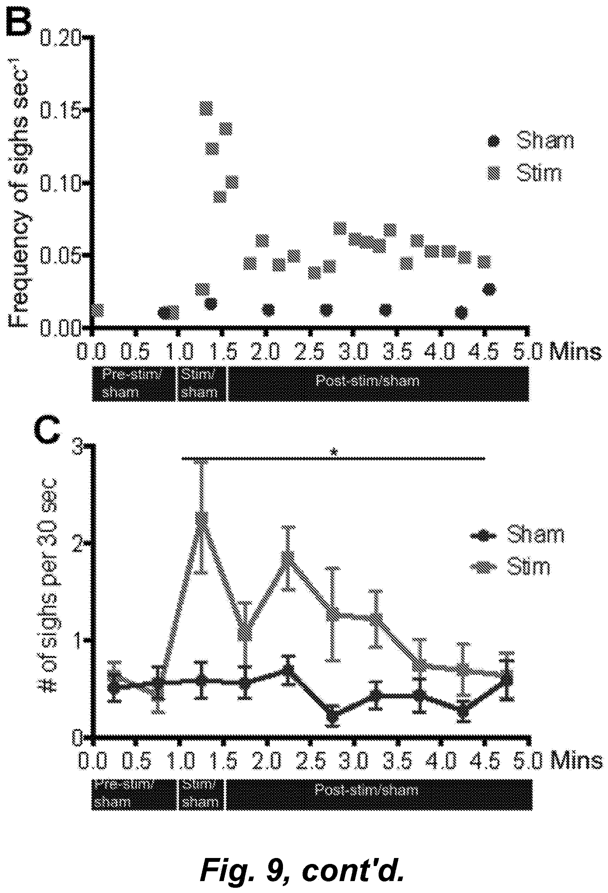

Figure 9, panel A, is a representative trace showing the recorded tidal volume (red trace) and smoothed tidal volume (black trace) of respiration pre-stimulation, during sham or during epidural stimulation and after sham or epidural stimulation. The peaks (see blue arrows) indicate sighs, and a representative sigh is shown in detail (black dash circle on the right). Fig. 9, panel B, shows a representation of the frequency of sighs per second depending on whether sham stimulation or epidural stimulation was used. Fig. 9, panel C, shows the number of sighs for every 30 seconds in all the mice tested (n=19) either pre-stimulation, during sham or during epidural stimulation and after sham or epidural stimulation. The number of sighs was greater during epidural stimulation compared to the pre-stimulation, baseline period (p = 0.002); significantly greater during epidural stimulation was delivered compared to the same period during sham stimulation (p = 0.0011, see asterisk); and sigh frequency remained significantly elevated for up to 3 min after epidural stimulation stopped compared to sham (p < 0.001, see asterisk).

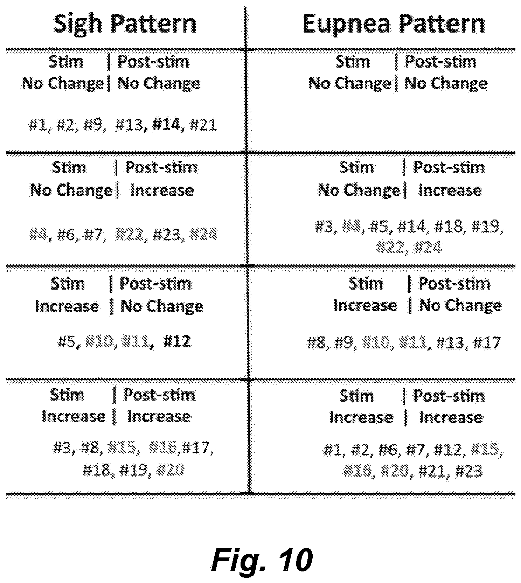

Figure 10 shows that sigh and eupnea response after stimulation is not correlated. The left side of the chart shows the pattern of sigh clustering based on the response to stimulation compared to pre-stimulation sigh baseline. The right side of the chart shows eupnea frequency clustering based on the response to epidural stimulation compared to the pre- stimulation eupnea baseline. Each animal appears on each side of the figure according to the pattern of sighing and eupneic responses, and those animals in which the pattern of changes in sigh and eupnea were similar during and after epidural stimulation are printed in bold, red text. The effects of epidural stimulation on the frequency of eupnea and sighs seemed to be uncorrelated in individual animals in that 16 of 24 animals had different sigh and eupneic responses patterns to epidural stimulation.
Figure 11, panels A-C, illustrates intraoperative human stimulation. Panel A shows the dorsal cervical cord from C3 to C6 with probe placed against dura of the cord for stimulation; here just left of the midline. Panel B shows a view of the dorsal/posterior medulla at the level of the cerebellar tonsils. The plane of the medullary surface can be probed to access the floor of the 4^{th} ventricle, the hypoglossal triangle, and the dorsal respiratory groups. Panel C shows that anterior cervical disc surgery can provide access to the ventral cord.
Figure 12 shows an overview of spinal respiratory stimulation. Anesthetized mice are monitored by pneumotach and EMG to monitor the respiratory rate. Heat map color code reflects an increase (yellow) or decrease (blue) in respiratory rate.
Figure 13 shows a summary of cervical respiratory stimulation in mouse to 30 Hz stimulation.
Figure 14 shows that a 30 Hz stimulation at C3/4 can induce respiration during deep anesthesia in humans.
Figure 15 shows that a 30 Hz stimulation at C3/4 can induce coordinated respiration during off-state in humans.
Figure 16 shows a representative respiratory response to 30Hz at C3/4 in humans.
Figure 17 shows responses to spinal stimulation during deep anesthesia in humans.
Figure 18 shows responses to spinal stimulation during light anesthesia in humans.
Figure 19, panels A-H, shows the respiratory responses to epidural stimulation at 30 Hz in human subjects. Epidural stimulation (30 Hz) was performed in the thoracic (panels A, B, E, F) and lumbar (panels C, D, G, H) locations. Stimulation resulted in significant increases in both tidal volume (panels A, E, C, G) and frequency (panels B, F, D, H).
Figure 20 illustrates parameters that can be varied using a transcutaneous stimulator (*e.g.*, a portable stimulator) as described herein. In certain embodiments the parameters that can be controlled/varied include stimulation frequency, stimulation amplitude, stimulation pulse width. In certain embodiments the carrier frequency, and/or carrier pulse width can be controlled. In certain embodiments the carrier signal can be turned off while leaving a stimulation signal on.
Figure 21 illustrates a schematic of one embodiment of a portable stimulator: An iphone or android platform can communicate by bluetooth or WiFi with the stimulator with the output signal as described in Figure 20.
Figure 22 illustrates a practical demonstration of the stimulator design.

### DETAILED DESCRIPTION

Respiration or breathing involves a complex network of circuits that is involved in central pattern generation (CPG) that spans the brainstem and cervical spinal cord to generate a respiratory rhythm. Sensory input from central and peripheral chemoreceptors (CO₂), lung stretch receptors naturally influence the firing pattern of the CPG. In the disease or injury state to the brainstem or spinal cord, the respiratory rhythm is compromised, likely due to the depressed state of the CPG.

Current technology in addressing the depressed respiratory state is to stimulate the diaphragm muscle which actively participates in inspiration by phrenic nerve stimulators. The issue with this approach is that the muscle will not react to changes in the activity state of the patient and only activates the diaphragm which participates in the inspiratory phase of respiration . To fully recapitulate normal breathing in the diseased states, the source of the problem within the central pattern generator(s) needs to be addressed.

In surgery, we have discovered the following which can be leveraged to restore normal breathing in traumatic or disease setting:
1. Stimulation with electrical current (optimal of frequency of 30-60 Hz) of the cervical, and/or thoracic, and/or lumbar spinal cord can modulate breathing (rate, rhythm, tidal volume);
2. Stimulation with electrical current (particularly at frequency of 5 Hz) of cervical, and/or thoracic, and/or lumbar nerve roots can modulate breathing (rate, rhythm, tidal volume);
3. Stimulation with electrical current can restore respiratory rhythm in a depressed respiratory state (*e.g*., opiate induced);
4. Combination of stimulation above can strongly influence and restore respiration in depressed respiratory state; and
5. Serotonin agonist medication such as buspirone can be used as tool to further activate the CPG associated with breathing.

In various embodiments stimulation of the above parameters of the various structures can be induced by epidural stimulation electrodes, non-invasive transcutaneous electrical stimulation, or magnetic stimulation.

Accordingly, in various embodiments, methods of improving, and/or regulation, and/or restoring respiration in a subject with a respiratory deficiency are provided. The methods typically involve neuromodulating the cervical, and/or thoracic, and/or lumbar spinal cord of the subject by administering transcutaneous stimulation to the cervical, and/or thoracic, and/or lumbar spinal cord or a region thereof at a frequency and intensity sufficient to restore respiration; and/or neuromodulating the spinal cord of the subject by administering epidural stimulation to spinal cord or a region thereof at a frequency and intensity sufficient to restore respiration; and/or neuromodulating the spinal cord of said subject with a magnetic stimulator at a frequency and intensity sufficient to restore respiration.

Unlike phrenic stimulation which directly stimulates the diaphragm muscle and does not recapitulate the afferent sensory feedback associated with respiration (*e.g*., CO₂, tidal volume, rate, *etc.*), the methods described herein that involve stimulation of nerve root and/or spinal cord, activate a respiratory drive that is responsive to normal feedback. It is believed more fully recapitulate normal breathing patterns. Additionally, the stimulation methods described herein can enable coughing, facilitate the opening of airways and facilitate swallowing.

It is believed there are no previous methods of activating the respiratory network to induce respiration in settings of decreased respiratory drive such as brain injury (stroke, traumatic brain injury, spinal cord injury), medication overdose, neurodegenerative disorders (ALS, Alzheimer's, Parkinson's, multiple sclerosis), among others. The methods described herein can be used to activate the respiratory network to wean patients from ventilators, or as respiratory "pacemakers" to maintain respiratory drive, therefore replacing ventilators. In the traumatic setting, this strategy can be used as a "respiratory defibrillator" (similar to a cardiac defibrillator) equivalent for respiration. The spinal cord can be thus be stimulated to activate the respiratory drive in a number of contexts where a subject has a respiratory deficiency (*e.g*., reduced respiratory drive).

In certain embodiments, the respiratory defibrillator can be integrated into an automatic external defibrillator (AED). An automated external defibrillator (AED) is a portable device that can check the heart rhythm and can send an electric shock to the heart to try to restore a normal rhythm. Where the respiratory defibrillator is integrated into an AED, the same device can be used to stimulate a heart rhythm and/or to stimulate or maintain respiration as necessary.

The methods described herein are typically for use with a mammal (*e.g*., a human, a mammal (*e.g.,* a non-human primate, equine, feline, canus, *etc*.) with a respiratory deficiency. Such respiratory deficiencies can arise in a number of contexts. For example, they can arise where a subject has a brain injury and/or a spinal cord injury. In the latter context, it is believed the methods described herein will be effective where the spinal cord injury is clinically classified as motor complete or motor incomplete. The methods also find use in cases of ischemic brain injury (*e.g.*, due to stroke, drowning or other oxygen deficiency, or acute trauma). It is also believed the methods will also find use there the respiratory deficiency is due to a neurodegenerative disorder (*e.g*., Parkinson's disease, Huntington's disease, Alzheimer's disease, amyotrophic lateral sclerosis (ALS), primary lateral sclerosis (PLS), dystonia, cerebral palsy, and the like). The methods also find use in cases of depressed respiration due to drug overdose.

It is also noted that in certain embodiments, the methods described herein can be used in conjunction with conventional respirators, *e.g*., to provide improved respiratory action or the methods can be used to substitute for or to wean a subject off of a traditional respirator.

In certain embodiments, the methods described herein can also be used in combination with the administration of one or more neuromodulatory drugs (*e.g.,* a monoaminergic agonist as described herein).

In certain embodiments the methods, and apparatus (*e.g*. systems) described herein can be used to wean a subject from a ventilator. By way of illustration, transcutaneous, epidural, and/or magnetic stimulation of respiration as described herein can be used to maintain respiration in a subject as they are removed from a respirator (ventilator) and/or after they have been removed from a ventilator, *e.g*., to facilitate respiration where there is an endogenous respiratory pattern, or to induce respiration where absent respirator or stimulation respiration would cease.

### Transcutaneous electrical stimulation.

In various embodiments transcutaneous electrical stimulation of the spinal cord (*e.g.,* one or more regions of the cervical spinal cord, and/or the thoracic spinal cord, and/or the lumbar spinal cord) is utilized to facilitate stimulation of and/or maintenance of respiratory function. In certain embodiments the epidural stimulation can be of one or more regions of the cervical spinal cord, and/or one or more regions of the thoracic spinal cord, and/or one or more regions of the lumbar spinal cord

The location of the electrode(s) and their stimulation parameters can be important in modulating the subject's respiration rate. Use of surface electrode(s), as described herein, facilitates selection or alteration of particular stimulation sites as well as the application of a wide variety of stimulation parameters. Additionally, surface stimulation can be used to optimize location for an implantable electrode or electrode array for epidural stimulation.

### Transcutaneous stimulation of the brainstem/suboccipital spinal cord or a region thereof.

In various embodiments, the methods described herein involve transcutaneous electrical stimulation of the brainstem and/or suboccipital region. This can be accomplished by application of one or more transcutaneous stimulation electrode, *e.g.,* as described herein, over the base of the skull and/or over the suboccipital region and the transcutaneous stimulation can be applied using the transcutaneous stimulation parameters described herein. As used herein the suboccipital region refers to a region of the neck bounded by the following three muscles of the suboccipital group of muscles: 1) Rectus capitis posterior major - above and medially; 2) Obliquus capitis superior - above and laterally; and 3) Obliquus capitis inferior - below and laterally.

### Transcutaneous electrical stimulation of the cervical spine or a region thereof

In certain embodiments, the methods described herein involve transcutaneous electrical stimulation of the cervical spine or a region of the cervical spine of the subject to restore or regulate respiration. Illustrative regions include, but are not limited to one or more regions straddling or spanning a region selected from the group consisting of C0-C1, C0-C2, C0-C3, C0-C4, C0-C5, C0-C6, C0-C7, C0-T1, C1-C1, C1-C2, C1-C3, C1-C4, C1-C7, C1-C6, C1-C7, C1-T1, C2-C2, C2-C3, C2-C4, C2-C5, C2-C6, C2-C7, C2-T1, C3-C3, C3-C4, C3-C5, C3-C6, C3-C7, C3-T1, C4-C4, C4-C5, C4-C6, C4-C7, C4-T1, C5-C5, C5-C6, C5-C7, C5-T1, C6-C6, C6-C7, C6-T1, C7-C7, and C7-T1.

In certain embodiments the transcutaneous stimulation is applied at a region comprising C2-C4 or a region therein. In certain embodiments the stimulation is applied at C3.

### Transcutaneous electrical stimulation of the thoracic spine or a region thereof

In various embodiments, the methods described herein additionally or alternatively involve transcutaneous electrical stimulation of the thoracic spine (*e.g*., spinal cord) or a region of the thoracic spine (spinal cord) of the subject to modulate and/or induce respiration. Illustrative regions include, but are not limited to, one or more regions straddling or spanning a region selected from the group consisting of T1-T1, T1-T2, T1-T3, T1-T4, T1-T5, T1-T6, T1-T7, T1-T8, T1-T9, T1-T10, T1-T11, T1-T12, T2-T2, T2-T3, T2-T4, T2-T5, T2-T6, T2-T7, T2-T8, T2-T9, T2-T10, T2-T11, T2-T12, T3-T3, T3-T4, T3-T5, T3-T6, T3-T7, T3-T8, T3-T9, T3-T10, T3-T11, T3-T12, T4-T4, T4-T5, T4-T6, T4-T7, T4-T8, T4-T9, T4-T10, T4-T11, T4-T12, T5-T5, T5-T6, T5-T7, T5-T8, T5-T9, T5-T10, T5-T11, T5-T12, T6-T6, T6-T7, T6-T8, T6-T9, T6-T10, T6-T11, T6-T12, T7-T7, T7-T8, T7-T9, T7-T10, T7-T11, T7-T12, T8-T8, T8-T9, T8-T10, T8-T11, T8-T12, T9-T9, T9-T10, T9-T11, T9-T12, T10-T10, T10-T11, T10-T12, T11-T11, T11-T12, and T12-T12.

### Transcutaneous electrical stimulation of the lumbar spine or a region thereof, and/or coccyx.

In various embodiments, the methods described herein additionally or alternatively involve transcutaneous electrical stimulation of the thoracic spine (*e.g*., spinal cord) or a region of the thoracic spine (spinal cord) of the subject to modulate and/or induce respiration. Illustrative regions include, but are not limited to, one or more regions straddling or spanning a region selected from the group consisting of L1-L1, L1-L2 , L1-L3, L1-L4, L1-L5, L1-S1, L1-S2, L1-S3, L1-S4, L1-S5, L2-L2 , L2-L3, L2-L4, L2-L5, L2-S1, L2-S2, L2-S3, L2-S4, L2-S5, L3-L3, L3-L4, L3-L5, L3-S1, L3-S2, L3-S3, L3-S4, L3-S5, L4-L4, L4-L5, L4-S1, L4-S2, L4-S3, L4-S4, L4-S5, L5-L5 , L5-S1, L5-S2, L5-S3, L5-S4, L5-S5, S1-S1, S1-S2, S1-S3, S1-S4, S1-S5, S2-S2, S2-S3, S2-S4, S2-S5, S3-S3, S3-S4, S3-S5, S4-S4, S4-S5, and S5-S6.

In certain embodiments, in addition, or as an alternative to the above-identified locations, transcutaneous stimulation can be applied to the coccyx, *e.g*., by application of one or more transcutaneous stimulation electrode(s) over the small, triangular bone at the base of the spinal column.

In certain embodiments the transcutaneous electrical stimulation is applied paraspinally over a lumbar region identified above or to a region thereof, *e.g.,* over a region spanning L2 to L3).

### Transcutaneous electrical stimulation parameters.

In certain embodiments the transcutaneous stimulation is at a frequency of at least about 1 Hz, or at least about 2 Hz, or at least about 3 Hz, or at least about 4 Hz, or at least about 5 Hz, or at least about 10 Hz, or at least about 20 Hz or at least about 30 Hz or at least about 40 Hz or at least about 50 Hz or at least about 60 Hz or at least about 70 Hz or at least about 80 Hz or at least about 90 Hz or at least about 100 Hz, or at least about 200 Hz, or at least about 300 Hz, or at least about 400 Hz, or at least about 500 Hz, or at least about 1 kHz, or at least about 1.5 kHz, or at least about 2 kHz, or at least about 2.5 kHz, or at least about 5 kHz, or at least about 10 kHz, or up to about 25 kHz, or up to about 50 kHz, or up to about 100 kHz.

In certain embodiments the transcutaneous stimulation is at a frequency ranging from about 1 Hz, or from about 2 Hz, or from about 3 Hz, or from about 4 Hz, or from about 5 Hz, or from about 10 Hz, or from about 10 Hz, or from about 10 Hz, up to about 500 Hz, or up to about 400 Hz, or up to about 300 Hz, or up to about 200 Hz up to about 100 Hz, or up to about 90 Hz, or up to about 80 Hz, or up to about 60 Hz, or up to about 40 Hz, or from about 3 Hz or from about 5 Hz up to about 80 Hz, or from about 5 Hz to about 60 Hz, or up to about 30 Hz. In certain embodiments the transcutaneous stimulation is at a frequency ranging from about 20 Hz or about 30 Hz to about 90 Hz or to about 100 Hz, to initiate respiration when no respiration pattern is present. In certain embodiments the transcutaneous stimulation is at a frequency ranging from about 5 Hz or about 10 Hz up to about 90 Hz or about 100 Hz, when a respiration pattern is present.

In certain embodiments the transcutaneous stimulation is applied at an intensity ranging from about 5 mA or about 10 mA up to about 500 mA, or from about 5 mA or about 10 mA up to about 400 mA, or from about 5 mA or about 10 mA up to about 300 mA, or from about 5 mA or about 10 mA up to about 200 mA, or from about 5 mA or about 10 mA to up about 150 mA, or from about 5 mA or about 10 mA up to about 50 mA, or from about 5 mA or about 10 mA up to about 100 mA, or from about 5 mA or about 10 mA up to about 80 mA, or from about 5 mA or about 10 mA up to about 60 mA, or from about 5 mA or about 10 mA up to about 50 mA.

In certain embodiments the transcutaneous stimulation is applied stimulation comprises pulses having a width that ranges from about 100 µs up to about 1 ms or up to about 800 µs, or up to about 600 µs, or up to about 500 µs, or up to about 400 µs, or up to about 300 µs, or up to about 200 µs, or up to about 100 µs, or from about 150 µs up to about 600 µs, or from about 200 µs up to about 500 µs, or from about 200 µs up to about 400 µs.

In certain embodiments the transcutaneous stimulation is at a frequency, pulse width, and amplitude sufficient to restore a resting respiration rate and at least 60%, or at least 70%, or at least 80%, or at least 90% of the subjects normal tidal volume.

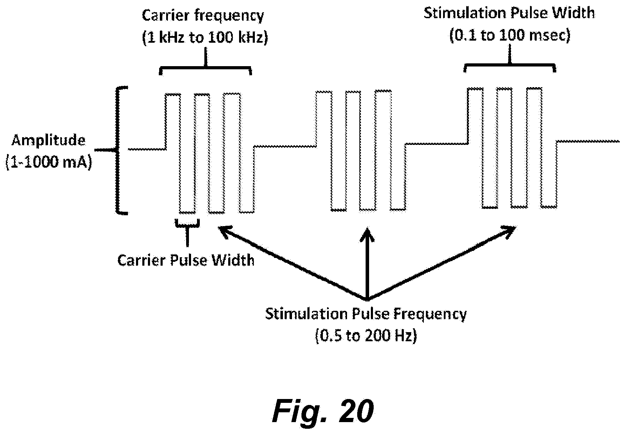

In certain embodiments the transcutaneous stimulation is superimposed on a high frequency carrier signal (see, e.g., Figure 20). In certain embodiments the high frequency carrier signal ranges from about 1 kHz, or about 3 kHz, or about 5 kHz, or about 8 kHz up to about 30 kHz, or up to about 20 kHz, or up to about 15 kHz. In certain embodiments the carrier signal is about 10 kHz. In certain embodiments the carrier frequency amplitude ranges from about 30 mA, or about 40 mA, or about 50 mA, or about 60 mA, or about 70 mA, or about 80 mA up to about 300 mA, or up to about 200 mA, or up to about 150 mA.

### Transcutaneous stimulation electrodes.

In certain embodiments the transcutaneous stimulation is applied using any of a number different types of electrodes. Such electrodes include, but are not limited to metal plate electrodes, carbon electrodes, textile electrodes, hydrogel electrodes, needle electrodes, and the like skin (*see, e.g.,* Keller & Kuhn (2008) J. Automatic Control., 18(2): 34-45). In various embodiments, the electrodes can be adhered using, *e.g*., tape or other adherent, or in other embodiments, the electrodes are self-adhering.

Metal plate electrodes include, but are not limited to metal plate electrodes covered by fabric tissue. Typically the metal plate is fabricated from a biocompatible material. Often stainless steel or silver/silver chloride electrodes are used. The fabric tissue can be cotton but is often a polymer textile material that has a certain degree of elasticity and doesn't wear out fast. Spongy materials have also been used and recommended (*see, e.g.,* Falk et al. (1983) N. Engl. J. Med. 309: 1166-1168). In certain embodiments, the fabric can be made conductive with water or electrode gel. It equally distributes the current over the skin in order to prevent skin burns. Care has to be taken that the electrode does not dry out. In the best case (if completely dry) such a dried out electrode isolates the metal plate from the skin. But while drying out, unequally distributed electrical fields under the electrodes may cause skin burns. The electrodes are typically fixed to the skin with elastic straps (*see, e.g.,* Ijezerman et al. (1996) J. Rehab. Sci. 9: 86-89).

Self-adhesive electrodes for transcutaneous stimulation use a gel to contact a conductive member with the subject's skin (*see, e.g.,* Keller & Kuhn (2008) J. Automatic Control., 18(2): 34-45). The electrode is typically built in a multi-layer configuration, consisting of multiple layers of hydrogel. The skin interface layer often includes an electrically conductive gel with relatively low peel strength for removably contacting the subject's skin. It has a wet feeling and can be removed relatively easily from the skin. In various illustrative, but non-limiting embodiments, the conductive gel is made from copolymers derived from polymerization, e.g. of acrylic acid and N-vinylpyrrolidone. In various illustrative embodiments, a second hydrogel layer connects the substrate (a low resistive material like carbon rubber or a wire mesh) with the skin hydrogel layer. This second conductive gel layer has a relatively high peel strength that provides good adhesion to the substrate.

In certain embodiments, carbon loaded silicon electrodes can be used (*see, e.g.,* Baker, D. R. McNeal, L. A. Benton, B. R. Bowman, and R. L. Waters, Neuromuscular electrical stimulation: a practical guide, 3 ed. USA: Rehabilitation Engineering Program, Los Amigos Research and Education Institute, Rancho Los Amigos Medical Center, 1993; Nathan (1989) J. Automatic Control, 18(2): 35-45; Patterson & Lockwood (1993) IEEE Trans. on Neural Systems and Rehabilitation, 1: 59-62; and the like).

In certain embodiments, the transcutaneous electrical stimulation can be applied via textile electrodes. In one illustrative, but non-limiting embodiment, the textile electrodes can consist of multiple fabric layers (*see, e.g.,* Keller, et al. (2006) Conf. Proc. IEEE Eng. Med. Biol. Soc. 1: 194-197). In certain embodiments, the fabric layer facing the skin holds embroidered electrode pads made of plasma coated metallized yarn. Because of the thin metal coating (*e.g.,* <25 nm coating particles obtained using a plasma process) the yarn keeps its textile properties and can be embroidered. Silver coatings proved to be most stable and survived 30 washings. A second layer contains the embroidered electrode wiring made from the same materials and was designed such that no short circuits are produced between the pads when stitched together (*Id*).

In certain embodiments, the transcutaneous electrical stimulation can be applied via one or more needle electrodes, *e.g*., as described in PCT Patent Pub No: WO 2017/024276 (PCT/US2016/045898). As described therein, needle electrodes comprise one or more commonly a plurality of electrically conductive solid microprojections (or where the needles are hollow, they are closed at the tip), where the needles (microprojections) have a tip dimension/diameter small enough to facilitate penetration of the stratum corneum on the skin (*e.g.,* less than about 10 µm), where the needles have a length greater than about 20 µm and where the electrically conductive solid needles are electrically coupled to one or more electrical leads. In one illustrative, but non-limiting embodiment, needles with tip size of several µm or smaller, and a shaft length of 50 µm or more can be used for transcutaneous electrical stimulation electrodes. In one illustrative, but non-limiting embodiment, a single electrode unit, consisting of 5x5 to 30x30 needles, is about one centimeter in diameter. Multiple electrode units can be further combined into an electrode array, *e.g*., when larger electrode areas are needed (for example, for the return/ground electrodes). The needle electrodes can provide low impedance transcutaneous stimulation without using a conductive gel or cream. In certain embodiments the needle electrodes comprise one or a plurality of electrically conductive needles, where the needles are solid, or wherein the needles are hollow and have a closed tip, where the needles having an average tip diameter less than about 10 µm and an average length greater than about 10 µm or greater than about 20 µm were the electrically conductive needles can be electrically coupled to one or more electrical leads. In certain embodiments, the needle electrode comprises at least about 10 needles, or at least about 15 needles, or at least about 20 needles, or at least about 25 needles, or at least about 30 needles, or at least about 40 needles, or at least about 50 needles, or at least about 100 needles, or at least about 200 needles, or at least about 300 needles, or at least about 400 needles, or at least about 500 needles, or at least about 600 needles, or at least about 700 needles, or at least about 800 needles, or at least about 900 needles, or at least about 1000 needles. In certain embodiments, the needle(s) comprising the needle electrode are of sufficient length to penetrate at least 70%, or at least 80%, or at least 90%), or at least 100%> through the stratum corneum of the skin when the electrode is attached to the surface of a human over the spinal cord. In certain embodiments, the needle(s)s are of a length that does not substantially penetrate subcutaneous tissue below the stratum corneum. In certain embodiments the average length of needle(s) comprising the needle electrode ranges from about 1 µm up to about 100 µm, or from about 1 µm up to about 80 µm, or from about 1 µm up to about 50 µm, or from about 1 µm up to about 30 µm, or from about 1 µm up to about 20 µm , or is at least about 30 µm, or at least about 40 µm, or at least about 50 µm, or at least about 60 µm, or at least about 70 µm. In certain embodiments the average length of the needle(s) is less than about 200 µm, or less than about 150 µm, or less than about 100 µm. In certain embodiments the average length of the needle(s) ranges from about 40 to about 60 µm.

In certain embodiments the average length of the needle(s) is about 50 µm. In certain embodiments the tip of the needle(s) ranges in diameter (or maximum cross-sectional dimension) from about 0.1 µm up to about 10 µm, or from about 0.5 µm up to about 6 µm, or from about 1 µm up to about 4 µm. In certain embodiments the average separation between two adjacent needles ranges from about 0.01 mm up to about 1 mm, or about 0.05 mm up to about 0.5 mm, or about 0.1 mm up to about 0.4 mm, or up to about 0.3 mm, or up to about 0.2 mm. In certain embodiments the average separation between two adjacent needles ranges from about 0.15 mm up to about 0.25 mm. In certain embodiments the needles are disposed in an area of about 1 cm² or less, or about 0.8 cm² or less, or about 0.6 cm² or less, or about 0.5 cm² or less, or about 0.4 cm² or less, or about 0.3 cm² or less, or about 0.2 cm² or less, or about 0.1 cm² or less. In certain embodiments the needles are disposed in an area of about 2 mm or about 3 mm, or about 4 mm, or about 5 mm, or about 6 mm, or about 7 mm or about 8 mm, or about 9 mm, or about 10 mm by about 2 mm or about 3 mm, or about 4 mm, or about 5 mm, or about 6 mm, or about 7 mm or about 8 mm, or about 9 mm, or about 10 mm. In certain embodiments the electrode comprises about 20 x about 20 needles in an area about 4 x 4 mm.

The foregoing electrodes for transcutaneous electrical stimulation are illustrative and non-limiting. Using the teaching provided herein, numerous other electrodes and/or electrode configurations will be available to one of skill in the art.

### Epidural stimulation

In various embodiments epidural stimulation of the spinal cord is utilized to facilitate stimulation of and/or maintenance of respiratory function. In certain embodiments the epidural stimulation can be of one or more regions of the cervical spinal cord, and/or one or more regions of the thoracic spinal cord, and/or one or more regions of the lumbar spinal cord

### Epidural stimulation of the brainstem/suboccipital spinal cord or a region thereof.

In various embodiments, the methods described herein involve epidural electrical stimulation of the brainstem and/or suboccipital region. As used herein the suboccipital region refers to a region of the neck bounded by the following three muscles of the suboccipital group of muscles: 1) Rectus capitis posterior major - above and medially; 2) Obliquus capitis superior - above and laterally; and 3) Obliquus capitis inferior - below and laterally. In various embodiments, stimulation can be via, *inter alia,* an implanted electrode or electrode array.

### Epidural stimulation of the cervical spine or a region thereof

In various embodiments, the methods described herein involve epidural electrical stimulation of the cervical spine (e.g., spinal cord) or a region of the cervical spine (spinal cord) of the subject to modulate and/or induce respiration. Illustrative regions include, but are not limited to, one or more regions straddling or spanning a region selected from the group consisting of C0-C1, C0-C2, C0-C3, C0-C4, C0-C5, C0-C6, C0-C7, C0-T1, C1-C1, C1-C2, C1-C3, C1-C4, C1-C7, C1-C6, C1-C7, C1-T1, C2-C2, C2-C3, C2-C4, C2-C5, C2-C6, C2-C7, C2-T1, C3-C3, C3-C4, C3-C5, C3-C6, C3-C7, C3-T1, C4-C4, C4-C5, C4-C6, C4-C7, C4-T1, C5-C5, C5-C6, C5-C7, C5-T1, C6-C6, C6-C7, C6-T1, C7-C7, and C7-T1.

In certain embodiments the epidural stimulation is applied paraspinally over a cervical region identified above (e.g., over vertebrae spanning C0 to C8 or a region thereof, *e.g.,* over a region spanning C2 to C4).

In certain embodiments the epidural stimulation is applied at a region comprising C2-C4 or a region therein. In certain embodiments the stimulation is applied at C3.

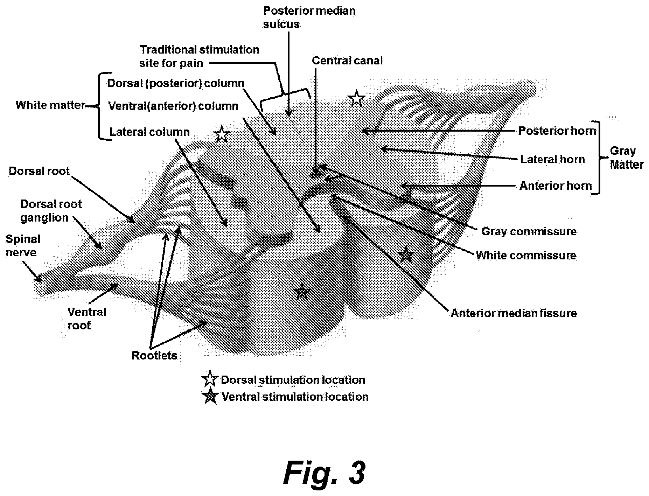

In certain embodiments the epidural stimulation is applied to the dorsal (posterior) column (see, e.g., Figure 3) and in certain embodiments to the lateral portion of the dorsal (posterior) column as shown in Figure 3.

In certain embodiments the epidural stimulation is alternatively or additionally applied to a dorsal root, and in certain embodiments to a dorsal root at the point of entry (see, e.g., Figure 3).

In certain embodiments the epidural stimulation is alternatively or additionally applied to a ventral (anterior) column and in certain embodiments to a lateral portion of the ventral column (see, e.g., Figure 3).

In certain embodiments the epidural stimulation is alternatively or additionally applied to a ventral root and in certain embodiments to a ventral root at the point of entry.

In certain embodiments the epidural stimulation to a ventral column and/or a ventral root speeds up respiration in a subject that is already breathing.

In various embodiments, the cervical epidural stimulation can be via, *inter alia,* an implanted electrode or electrode array and/or by use of one or more needle electrodes.

### Epidural stimulation of the thoracic spine or a region thereof

In various embodiments, the methods described herein additionally or alternatively involve epidural electrical stimulation of the thoracic spine (e.g., spinal cord) or a region of the thoracic spine (spinal cord) of the subject to modulate and/or induce respiration. Illustrative regions include, but are not limited to, one or more regions straddling or spanning a region selected from the group consisting of T1-T1, T1-T2, T1-T3, T1-T4, T1-T5, T1-T6, T1-T7, T1-T8, T1-T9, T1-T10, T1-T11, T1-T12, T2-T2, T2-T3, T2-T4, T2-T5, T2-T6, T2-T7, T2-T8, T2-T9, T2-T10, T2-T11, T2-T12, T3-T3, T3-T4, T3-T5, T3-T6, T3-T7, T3-T8, T3-T9, T3-T10, T3-T11, T3-T12, T4-T4, T4-T5, T4-T6, T4-T7, T4-T8, T4-T9, T4-T10, T4-T11, T4-T12, T5-T5, T5-T6, T5-T7, T5-T8, T5-T9, T5-T10, T5-T11, T5-T12, T6-T6, T6-T7, T6-T8, T6-T9, T6-T10, T6-T11, T6-T12, T7-T7, T7-T8, T7-T9, T7-T10, T7-T11, T7-T12, T8-T8, T8-T9, T8-T10, T8-T11, T8-T12, T9-T9, T9-T10, T9-T11, T9-T12, T10-T10, T10-T11, T10-T12, T11-T11, T11-T12, and T12-T12.

In certain embodiments the epidural stimulation is applied paraspinally over a thoracic region identified above (*e.g*., over vertebrae spanning T1-T12, or a region thereof, *e.g.,* over a region spanning T2 to T3).

In certain embodiments the epidural stimulation is applied to the dorsal (posterior) column and in certain embodiments to the lateral portion of the dorsal (posterior) column.

In certain embodiments the epidural stimulation is alternatively or additionally applied to a dorsal root, and in certain embodiments to a dorsal root at the point of entry.

In certain embodiments the epidural stimulation is alternatively or additionally applied to a ventral (anterior) column and in certain embodiments to a lateral portion of the ventral column.

In certain embodiments the epidural stimulation is alternatively or additionally applied to a ventral root and in certain embodiments to a ventral root at the point of entry.

In certain embodiments the epidural stimulation to a ventral column and/or a ventral root speeds up respiration in a subject that is already breathing.

In various embodiments, the thoracic epidural stimulation can be via, *inter alia,* an implanted electrode or electrode array and/or by use of one or more needle electrodes.

### Epidural stimulation of the lumbar spine or a region thereof.

In various embodiments, the methods described herein additionally or alternatively involve epidural electrical stimulation of the thoracic spine (*e.g.*, spinal cord) or a region of the thoracic spine (spinal cord) of the subject to modulate and/or induce respiration. Illustrative regions include, but are not limited to, one or more regions straddling or spanning a region selected from the group consisting of L1-L1, L1-L2 , L1-L3, L1-L4, L1-L5, L1-S1, L1-S2, L1-S3, L1-S4, L1-S5, L2-L2 , L2-L3, L2-L4, L2-L5, L2-S1, L2-S2, L2-S3, L2-S4, L2-S5, L3-L3, L3-L4, L3-L5, L3-S1, L3-S2, L3-S3, L3-S4, L3-S5, L4-L4, L4-L5, L4-S1, L4-S2, L4-S3, L4-S4, L4-S5, L5-L5 , L5-S1, L5-S2, L5-S3, L5-S4, L5-S5, S1-S1, S1-S2, S1-S3, S1-S4, S1-S5, S2-S2, S2-S3, S2-S4, S2-S5, S3-S3, S3-S4, S3-S5, S4-S4, S4-S5, and S5-S6.

In certain embodiments the epidural stimulation is applied paraspinally over a lumbar region identified above or to a region thereof, *e.g.,* over a region spanning L2 to L3).

In certain embodiments the epidural stimulation is applied to the dorsal (posterior) column (and in certain embodiments to the lateral portion of the dorsal (posterior) column.

In certain embodiments the epidural stimulation is alternatively or additionally applied to a dorsal root, and in certain embodiments to a dorsal root at the point of entry.

In certain embodiments the epidural stimulation is alternatively or additionally applied to a ventral (anterior) column and in certain embodiments to a lateral portion of the ventral column.

In certain embodiments the epidural stimulation is alternatively or additionally applied to a ventral root and in certain embodiments to a ventral root at the point of entry.

In certain embodiments the epidural stimulation to a ventral column and/or a ventral root speeds up respiration in a subject that is already breathing.

In various embodiments, the lumbar epidural stimulation can be via, *inter alia,* an implanted electrode or electrode array and/or by use of one or more needle electrodes.

### Epidural stimulation parameters.

In certain embodiments, the epidural stimulation is at a frequency of at least about 1 Hz, or at least about 2 Hz, or at least about 3 Hz, or at least about 4 Hz, or at least about 5 Hz, or at least about 10 Hz, or at least about 20 Hz or at least about 30 Hz or at least about 40 Hz or at least about 50 Hz or at least about 60 Hz or at least about 70 Hz or at least about 80 Hz or at least about 90 Hz or at least about 100 Hz, or at least about 200 Hz, or at least about 300 Hz, or at least about 400 Hz, or at least about 500 Hz, or at least about 1 kHz, or at least about 1.5 kHz, or at least about 2 kHz, or at least about 2.5 kHz, or at least about 5 kHz, or at least about 10 kHz, or up to about 25 kHz, or up to about 50 kHz, or up to about 100 kHz.

In certain embodiments, the epidural stimulation is at a frequency ranging from about 1 Hz, or from about 2 Hz, or from about 3 Hz, or from about 4 Hz, or from about 5 Hz, or from about 10 Hz, or from about 10 Hz, or from about 10 Hz, up to about 500 Hz, or up to about 400 Hz, or up to about 300 Hz, or up to about 200 Hz up to about 100 Hz, or up to about 90 Hz, or up to about 80 Hz, or up to about 60 Hz, or up to about 40 Hz, or from about 3 Hz or from about 5 Hz up to about 80 Hz, or from about 5 Hz to about 60 Hz, or up to about 30 Hz.

In certain embodiments, the epidural stimulation is at a frequency ranging from about 20 Hz or about 30 Hz to about 90 Hz or to about 100 Hz, to initiate respiration when no respiration pattern is present.

In certain embodiments, the epidural stimulation is at a frequency ranging ranging from about 5 Hz or about 10 Hz up to about 90 Hz or about 100 Hz, when a respiration pattern is present.

In certain embodiments, the epidural stimulation is at an amplitude ranging from 0.5 mA, or from about 1 mA, or from about 2 mA, or from about 3 mA, or from about 4 mA, or from about 5 mA up to about 50 mA, or up to about 30 mA, or up to about 20 mA, or up to about 15 mA, or from about 5 mA to about 20 mA, or from about 5 mA up to about 15 mA.

In certain embodiments, the epidural stimulation is with pulses having a pulse width ranging from about 100 µs up to about 1 ms or up to about 800 µs, or up to about 600 µs, or up to about 500 µs, or up to about 400 µs, or up to about 300 µs, or up to about 200 µs, or up to about 100 µs, or from about 150 µs up to about 600 µs, or from about 200 µs up to about 500 µs, or from about 200 µs up to about 400 µs.

In certain embodiments, the epidural stimulation is at a frequency and amplitude sufficient to modulate and/or restore a resting (or active depending on context) respiration rate and at least 60%, or at least 70%, or at least 80%, or at least 90%, or at least 95%, or at least 98% of the subjects normal tidal volume.

In certain embodiments, the epidural stimulation is applied via a permanently implanted electrode array (*e.g*., a typical density electrode array, a high density electrode array, *etc*.).

In certain embodiments, the epidural electrical stimulation is administered via a high-density epidural stimulating array (*e.g.,* as described in PCT Publication No: WO/2012/094346 (PCT/US2012/020112). In certain embodiments, the high-density electrode arrays are prepared using microfabrication technology to place numerous electrodes in an array configuration on a flexible substrate. In some embodiments, epidural array fabrication methods for retinal stimulating arrays can be used in the methods described herein (see, *e.g.,* Maynard (2001) Annu. Rev. Biomed. Eng., 3: 145-168; Weiland and Humayun (2005) IEEE Eng. Med. Biol. Mag., 24(5): 14-21, and U.S. Patent Publications 2006/0003090 and 2007/0142878). In various embodiments, the stimulating arrays comprise one or more biocompatible metals (*e.g.,* gold, platinum, chromium, titanium, iridium, tungsten, and/or oxides and/or alloys thereof) disposed on a flexible material. Flexible materials can be selected from parylene A, parylene C, parylene AM, parylene F, parylene N, parylene D, silicon, other flexible substrate materials, or combinations thereof. Parylene has the lowest water permeability of available microfabrication polymers, is deposited in a uniquely conformal and uniform manner, has previously been classified by the FDA as a United States Pharmacopeia (USP) Class VI biocompatible material (enabling its use in chronic implants) (Wolgemuth, Medical Device and Diagnostic Industry, 22(8): 42-49 (2000)), and has flexibility characteristics (Young's modulus ~4 GPa (Rodger and Tai (2005) IEEE Eng. Med. Biology, 24(5): 52-57)), lying in between those of PDMS (often considered too flexible) and most polyimides (often considered too stiff). Finally, the tear resistance and elongation at break of parylene are both large, minimizing damage to electrode arrays under surgical manipulation. The preparation and parylene microelectrode arrays suitable for use in the epidural stimulation methods described herein is described in PCT Publication No: WO/2012/100260 (PCT/US2012/022257). Another suitable microelectrode array is the NEUROPORT^{®} microelectrode array (Cyberkinetics Neurotechnology Systems Inc., Boston, MA) which consists of 96 platinum microelectrodes, arranged in a 10 × 10 array without electrodes at the corners, affixed to a 4 mm² silicon base.

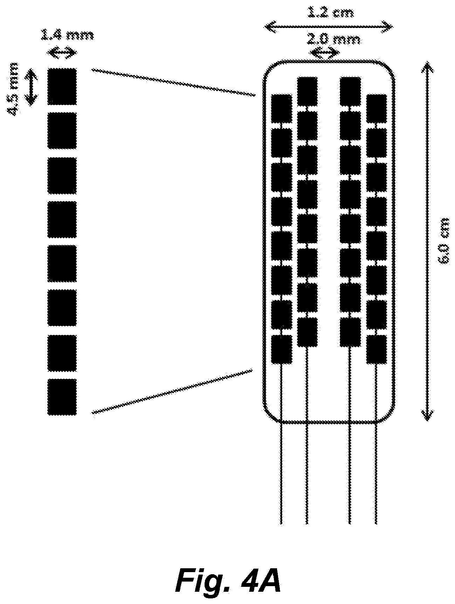

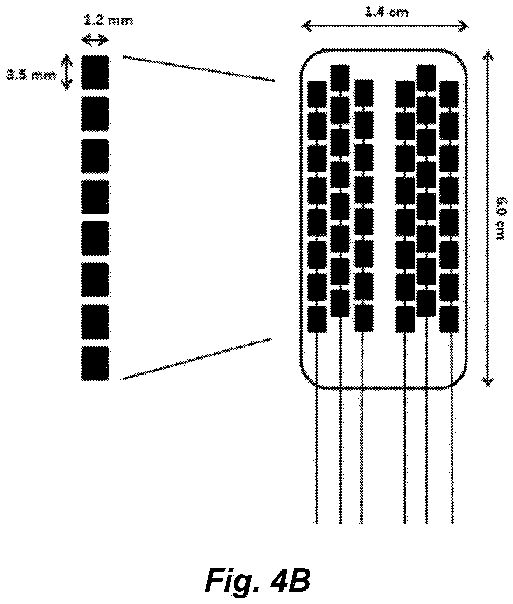

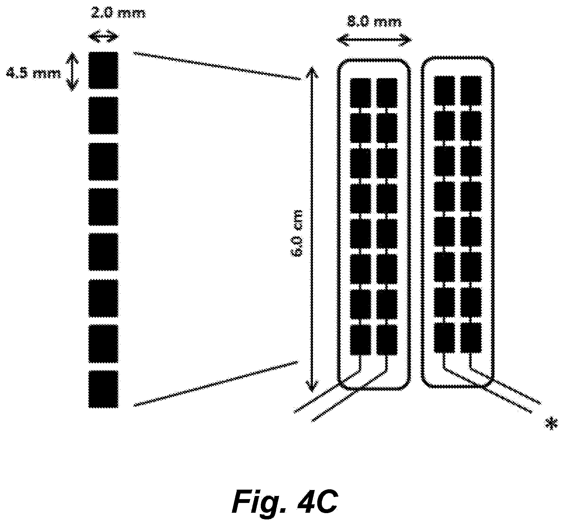

In certain illustrative, but non-limiting, embodiments an electrode array is utilized that has a configuration that provides a 32 channel dorsal respiration electrode type A, *e.g*., substantially as illustrated in Figure 4A. In certain illustrative, but non-limiting, embodiments an electrode array is utilized that has a configuration that provides a configuration that is a 48 channel dorsal respiration electrode type B, *e.g*., substantially as illustrated in Figure 4B. In certain illustrative, but non-limiting, embodiments an electrode array is utilized that has a configuration that provides an 8 channel ventral respiration dual electrode type C, *e.g*., substantially as illustrated in Figure 4C. In certain embodiments the electrode array has an inferolateral exiting electrode tail).

The electrode array may be implanted using any of a number of methods (*e.g*., a laminectomy procedure) well known to those of skill in the art. For example, in some embodiments, electrical energy is delivered through electrodes positioned external to the dura layer surrounding the spinal cord. Stimulation on the surface of the cord (subdurally) is also contemplated, for example, stimulation may be applied to the dorsal columns as well as to the dorsal root entry zone. In certain embodiments the electrodes are carried by two primary vehicles: a percutaneous lead and a laminotomy lead. Percutaneous leads can typically comprise two or more, spaced electrodes (*e.g*., equally spaced electrodes), that are placed above the dura layer, *e.g*., through the use of a Touhy-like needle. For insertion, the Touhy-like needle can be passed through the skin, between desired vertebrae, to open above the dura layer. An example of an eight-electrode percutaneous lead is an OCTRODE^{®} lead manufactured by Advanced Neuromodulation Systems, Inc.

Laminotomy leads typically have a paddle configuration and typically possess a plurality of electrodes (for example, two, four, eight, sixteen. 24, or 32) arranged in one or more columns. An example of an eight-electrode, two column laminotomy lead is a LAMITRODE^{®} 44 lead manufactured by Advanced Neuromodulation Systems, Inc. In certain embodiments the implanted laminotomy leads are transversely centered over the physiological midline of a subject. In such position, multiple columns of electrodes are well suited to administer electrical energy on either side of the midline to create an electric field that traverses the midline. A multi-column laminotomy lead enables reliable positioning of a plurality of electrodes, and in particular, a plurality of electrode rows that do not readily deviate from an initial implantation position.

Laminotomy leads are typically implanted in a surgical procedure. The surgical procedure, or partial laminectomy, typically involves the resection and removal of certain vertebral tissue to allow both access to the dura and proper positioning of a laminotomy lead. The laminotomy lead offers a stable platform that is further capable of being sutured in place.

In the context of conventional spinal cord stimulation, the surgical procedure, or partial laminectomy, can involve the resection and removal of certain vertebral tissue to allow both access to the dura and proper positioning of a laminotomy lead. Depending on the position of insertion, however, access to the dura may only require a partial removal of the ligamentum flavum at the insertion site. In certain embodiments, two or more laminotomy leads are positioned within the epidural space of C1-C7 as identified above. The leads may assume any relative position to one another.

In certain embodiments the electrode array is disposed on the nerve roots and/or the ventral surface. Electrode arrays can be inserted into the ventral and/or nerve root area via a laminotomy procedure.

In various embodiments, the arrays are operably linked to control circuitry that permits selection of electrode(s) to activate/stimulate and/or that controls frequency, and/or pulse width, and/or amplitude of stimulation. In various embodiments, the electrode selection, frequency, amplitude, and pulse width are independently selectable, *e.g*., at different times, different electrodes can be selected. At any time, different electrodes can provide different stimulation frequencies and/or amplitudes. In various embodiments, different electrodes or all electrodes can be operated in a monopolar mode and/or a bipolar mode, using constant current or constant voltage delivery of the stimulation. In certain embodiments time-varying current and/or time-varying voltage may be utilized.

In certain embodiments, the electrodes can also be provided with implantable control circuitry and/or an implantable power source. In various embodiments, the implantable control circuitry can be programmed/reprogrammed by use of an external device (*e.g*., using a handheld device that communicates with the control circuitry through the skin). The programming can be repeated as often as necessary.

The epidural electrode stimulation systems described herein are intended to be illustrative and non-limiting. Using the teachings provided herein, alternative epidural stimulation systems and methods will be available to one of skill in the art.

### Subdural stimulation.

In certain embodiments respiration can be stimulated by the application of subdural stimulation using the various stimulation parameters described herein. Typically, subdural stimulation is accomplished by the implantation of subdural electrodes, although in certain instances the use of one or more needle electrodes is contemplated.

The fabrication and use of subdural electrodes is well known to those of skill in the art, particularly in the treatment of epilepsy (*see, e.g.,* Lesser et al. (2010) Clin. Neurophysiol., 121(9): 1376-1392; Voorhies & Cohen-Gadol (2013) Surg. Neurol. Int. 4: 98; and the like).

Using the teaching provided herein subdural electrodes and implantation methods can readily be adapted to induce and/or facilitate respiration as described herein.

### Magnetic stimulation.

In certain embodiments magnetic stimulators can be also be used for stimulation of the cervical spinal cord, and/or the thoracic spinal cord, and/or the lumbar spinal cord to modulate/induce, and/or restore respiration. Magnetic spinal cord stimulation is achieved by generating a rapidly changing magnetic field to induce a current at the nerve(s) of interest (*e.g*., the spinal cord). Effective stimulation typically utilizes current transients of about 10⁸ *A*/*s* or greater discharged through a stimulating coil. The discharge current flowing through the stimulating coil generates magnetic lines of force. As the lines of force cut through tissue, a current is generated in that tissue, whether skin, bone, muscle or neural; if the induced current is of sufficient amplitude and duration such that the cell membrane is depolarized, neuromuscular tissue will be stimulated in the same manner as conventional electrical stimulation.

### Magnetic stimulation of the brainstem/suboccipital spinal cord or a region thereof.

In various embodiments, the methods described herein involve magnetic stimulation of the brainstem and/or suboccipital region. This can be accomplished by application of a magnetic stimulator (*e.g*., a magnetic stimulator wand) to the suboccipital region of the neck and stimulation can be according to any of the magnetic stimulation parameters described herein.

In certain embodiments stimulation can be accomplished by transcranial magnetic stimulation applied, *e.g.,* to the base of the skull.

### Magnetic Stimulation of the cervical spinal cord.

In various embodiments, the methods described herein involve magnetic stimulation of the cervical spine or a region of the cervical spine of the subject to modulate and/or induce respiration. Illustrative regions include, but are not limited to, one or more regions straddling or spanning a region selected from the group consisting of C0-C1, C0-C2, C0-C3, C0-C4, C0-C5, C0-C6, C0-C7, C0-T1, C1-C1, C1-C2, C1-C3, C1-C4, C1-C7, C1-C6, C1-C7, C1-T1, C2-C2, C2-C3, C2-C4, C2-C5, C2-C6, C2-C7, C2-T1, C3-C3, C3-C4, C3-C5, C3-C6, C3-C7, C3-T1, C4-C4, C4-C5, C4-C6, C4-C7, C4-T1, C5-C5, C5-C6, C5-C7, C5-T1, C6-C6, C6-C7, C6-T1, C7-C7, and C7-T1.

### Magnetic Stimulation of the thoracic spinal cord.

In various embodiments, the methods described herein involve magnetic stimulation of the thoracic spine or a region of the thoracic spine of the subject to modulate and/or induce respiration. Illustrative regions include, but are not limited to, one or more regions straddling or spanning a region selected from the group consisting of T1-T1, T1-T2, T1-T3, T1-T4, T1-T5, T1-T6, T1-T7, T1-T8, T1-T9, T1-T10, T1-T11, T1-T12, T2-T2, T2-T3, T2-T4, T2-T5, T2-T6, T2-T7, T2-T8, T2-T9, T2-T10, T2-T11, T2-T12, T3-T3, T3-T4, T3-T5, T3-T6, T3-T7, T3-T8, T3-T9, T3-T10, T3-T11, T3-T12, T4-T4, T4-T5, T4-T6, T4-T7, T4-T8, T4-T9, T4-T10, T4-T11, T4-T12, T5-T5, T5-T6, T5-T7, T5-T8, T5-T9, T5-T10, T5-T11, T5-T12, T6-T6, T6-T7, T6-T8, T6-T9, T6-T10, T6-T11, T6-T12, T7-T7, T7-T8, T7-T9, T7-T10, T7-T11, T7-T12, T8-T8, T8-T9, T8-T10, T8-T11, T8-T12, T9-T9, T9-T10, T9-T11, T9-T12, T10-T10, T10-T11, T10-T12, T11-T11, T11-T12, and T12-T12..

### Magnetic Stimulation of the lumbar spinal cord and/or coccyx.

In various embodiments, the methods described herein involve magnetic stimulation of the thoracic spine or a region of the thoracic spine of the subject to modulate and/or induce respiration. Illustrative regions include, but are not limited to, one or more regions straddling or spanning a region selected from the group consisting of of L1-L1, L1-L2 , L1-L3, L1-L4, L1-L5, L1-S1, L1-S2, L1-S3, L1-S4, L1-S5, L2-L2 , L2-L3, L2-L4, L2-L5, L2-S1, L2-S2, L2-S3, L2-S4, L2-S5, L3-L3, L3-L4, L3-L5, L3-S1, L3-S2, L3-S3, L3-S4, L3-S5, L4-L4, L4-L5, L4-S1, L4-S2, L4-S3, L4-S4, L4-S5, L5-L5 , L5-S1, L5-S2, L5-S3, L5-S4, L5-S5, S1-S1, S1-S2, S1-S3, S1-S4, S1-S5, S2-S2, S2-S3, S2-S4, S2-S5, S3-S3, S3-S4, S3-S5, S4-S4, S4-S5, and S5-S6.

In certain embodiments, in addition, or as an alternative to the above-identified locations, stimulation can be applied to the coccyx, *e.g*., by application of a transcutaneous stimulation electrode to the coccyx, i.e. over the small, triangular bone at the base of the spinal column.

### Magnetic stimulation parameters.

As noted above, magnetic stimulators can be used for stimulation of the spinal cord to modulate/induce, and/or restore respiration. Since the magnetic field strength falls off with the square of the distance from the stimulating coil, the stimulus strength is at its highest close to the coil surface. The stimulation characteristics of the magnetic pulse, such as depth of penetration, strength and accuracy, depend on the rise time, peak electrical energy transferred to the coil and the spatial distribution of the field. The rise time and peak coil energy are governed by the electrical characteristics of the magnetic stimulator and stimulating coil, whereas the spatial distribution of the induced electric field depends on the coil geometry and the anatomy of the region of induced current flow.

In various embodiments the magnetic nerve stimulator will produce a field strength up to about 10 tesla, or up to about 8 tesla, or up to about 6 tesla, or up to about 5 tesla, or up to about 4 tesla, or up to about 3 tesla, or up to about 2 tesla, or up to about 1 tesla. In certain embodiments the nerve stimulator produces pulses with a duration from about 100 µs up to about 10 ms, or from about 100 µs up to about 1 ms.

In certain embodiments the magnetic stimulation is at a frequency of at least about 1 Hz, or at least about 2 Hz, or at least about 3 Hz, or at least about 4 Hz, or at least about 5 Hz, or at least about 10 Hz, or at least about 20 Hz or at least about 30 Hz or at least about 40 Hz or at least about 50 Hz or at least about 60 Hz or at least about 70 Hz or at least about 80 Hz or at least about 90 Hz or at least about 100 Hz, or at least about 200 Hz, or at least about 300 Hz, or at least about 400 Hz, or at least about 500 Hz.

In certain embodiments the magnetic stimulation is at a frequency ranging from about 1 Hz, or from about 2 Hz, or from about 3 Hz, or from about 4 Hz, or from about 5 Hz, or from about 10 Hz, or from about 10 Hz, or from about 10 Hz, up to about 500 Hz, or up to about 400 Hz, or up to about 300 Hz, or up to about 200 Hz up to about 100 Hz, or up to about 90 Hz, or up to about 80 Hz, or up to about 60 Hz, or up to about 40 Hz, or from about 3 Hz or from about 5 Hz up to about 80 Hz, or from about 5 Hz to about 60 Hz, or up to about 30 Hz.

In certain embodiments the magnetic stimulation is at a frequency ranging from about 20 Hz or about 30 Hz to about 90 Hz or to about 100 Hz, to initiate respiration when no respiration pattern is present.

In certain embodiments the magnetic stimulation is at a frequency ranging from about 5 Hz or about 10 Hz up to about 90 Hz or about 100 Hz, when a respiration pattern is present.

In certain embodiments, the magnetic stimulation is at a frequency and amplitude sufficient to modulate and/or restore a resting (or active depending on context) respiration rate and at least 60%, or at least 70%, or at least 80%, or at least 90%, or at least 95%, or at least 98% of the subjects normal tidal volume.

It will be recognized that in various embodiments, transcutaneous electrical stimulation and/or epidural electrical stimulation, and/or magnetic stimulation can be applied at two or more locations sequentially or simultaneously. In certain embodiments stimulation at one site can be transcutaneous electrical stimulation while at another site can be by epidural and/or magnetic stimulation. In certain embodiments the stimulation comprises one or more combinations of stimulation modalities such as epidural and transcutaneous, epidural and magnetic, transcutaneous and magnetic, and epidural and transcutaneous and magnetic.

### Stimulators and Stimulation Systems.

### Electrical stimulators.

Any present or future developed stimulation system capable of providing an electrical signal to one or more regions of the spinal cord may be used in accordance with the teachings provided herein. Electrical stimulation systems (*e.g*., pulse generator(s)) can be used with both transcutaneous stimulation and epidural stimulation.

In various embodiments, the system may comprise an external pulse generator for use with either a transcutaneous stimulation system or an epidural system. In other embodiments the system may comprise an implantable pulse generator to produce a number of stimulation pulses that are sent to a region in proximity to the spinal cord by insulated leads coupled to the spinal cord by one or more electrodes and/or an electrode array to provide epidural stimulation. In certain embodiments the one or more electrodes or one or more electrodes comprising the electrode array may be attached to separate conductors included within a single lead. Any known or future developed lead useful for applying an electrical stimulation signal in proximity to a subject's spinal cord may be used. For example, the leads may be conventional percutaneous leads, such as PISCES^{®} model 3487A sold by Medtronic, Inc. In some embodiments, it may be desirable to employ a paddle-type lead.

Any known or future developed external or implantable pulse generator may be used in accordance with the teachings provided herein. For example, one internal pulse generator may be an ITREL^{®} II or Synergy pulse generator available from Medtronic, Inc, Advanced Neuromodulation Systems, Inc.'s GENESIS^{™} pulse generator, or Advanced Bionics Corporation's PRECISION^{™} pulse generator. One of skill in the art will recognize that the above-mentioned pulse generators may be advantageously modified to modulate respiration in accordance with the teachings provided herein.

In certain embodiments systems can employ a programmer coupled via a conductor to a radio frequency antenna. This system permits attending medical personnel to select the various pulse output options after implant using radio frequency communications. While, in certain embodiments, the system employs fully implanted elements, systems employing partially implanted elements may also be used in accordance with the teachings provided herein.

In one illustrative, but non-limiting system, a control module is operably coupled to a signal generation module and instructs the signal generation module regarding the signal to be generated. For example, at any given time or period of time, the control module may instruct the signal generation module to generate an electrical signal having a specified pulse width, frequency, intensity (current or voltage), *etc.* The control module may be preprogrammed prior to implantation or receive instructions from a programmer (or another source) through any known or future developed mechanism, such as telemetry. The control module may include or be operably coupled to memory to store instructions for controlling the signal generation module and may contain a processor for controlling which instructions to send to signal generation module and the timing of the instructions to be sent to signal generation module.

In certain embodiments, the controller alters and/or modulates respiration adjusting the respiration rate and/or tidal volume in response to the subject's heart rate and/or in response to variations in the measured respiration rate and/or tidal volume.

In various embodiments, leads are operably coupled to signal generation module such that a stimulation pulse generated by signal generation module may be delivered via electrodes.

While in certain embodiments, two leads are utilized, it will be understood that any number of one or more leads may be employed. In addition, it will be understood that any number of one or more electrodes per lead may be employed. Stimulation pulses are applied to electrodes (which typically are cathodes) with respect to a return electrode (which typically is an anode) to induce a desired area of excitation of electrically excitable tissue in a region of the spine. A return electrode such as a ground or other reference electrode can be located on same lead as a stimulation electrode. However, it will be understood that a return electrode may be located at nearly any location, whether in proximity to the stimulation electrode or at a more remote part of the body, such as at a metallic case of a pulse generator. It will be further understood that any number of one or more return electrodes may be employed. For example, there can be a respective return electrode for each cathode such that a distinct cathode/anode pair is formed for each cathode.

In various embodiments, the independent electrodes or electrodes of electrode arrays are operably linked to control circuitry that permits selection of electrode(s) to activate/stimulate and/or controls frequency, and/or pulse width, and/or amplitude of stimulation. In various embodiments, the electrode selection, frequency, amplitude, and pulse width are independently selectable, *e.g*., at different times, different electrodes can be selected. At any time, different electrodes can provide different stimulation frequencies and/or amplitudes. In various embodiments, different electrodes or all electrodes can be operated in a monopolar mode and/or a bipolar mode, using, *e.g*., constant current or constant voltage delivery of the stimulation.

In one illustrative but non-limiting system a control module is operably coupled to a signal generation module and instructs the signal generation module regarding the signal to be generated. For example, at any given time or period of time, the control module may instruct the signal generation module to generate an electrical signal having a specified pulse width, frequency, intensity (current or voltage), *etc.* The control module may be preprogrammed prior to use or receive instructions from a programmer (or another source). Thus, in certain embodiments the pulse generator/controller is configurable by software and the control parameters may be programmed/entered locally, or downloaded as appropriate/necessary from a remote site.

In certain embodiments the pulse generator/controller may include or be operably coupled to memory to store instructions for controlling the stimulation signal(s) and may contain a processor for controlling which instructions to send for signal generation and the timing of the instructions to be sent.

While in certain embodiments, two leads are utilized to provide transcutaneous or epidural stimulation, it will be understood that any number of one or more leads may be employed. In addition, it will be understood that any number of one or more electrodes per lead may be employed. Stimulation pulses are applied to electrodes (which typically are cathodes) with respect to a return electrode (which typically is an anode) to induce a desired area of excitation of electrically excitable tissue in one or more regions of the spine. A return electrode such as a ground or other reference electrode can be located on same lead as a stimulation electrode. However, it will be understood that a return electrode may be located at nearly any location, whether in proximity to the stimulation electrode or at a more remote part of the body, such as at a metallic case of a pulse generator. It will be further understood that any number of one or more return electrodes may be employed. For example, there can be a respective return electrode for each cathode such that a distinct cathode/anode pair is formed for each cathode.

In certain embodiments the controller component of the electrical stimulator is configured to receive signals from a heart rate monitor and/or respiration monitor and to adjust respiration parameters in response to changes in heart rate and/or respiration patterns.

### Portable stimulator for transcutaneous electrical stimulation of the spinal cord.

In certain embodiments a portable stimulator is provided for transcutaneous spinal cord stimulation. In various embodiments the portable stimulator is miniaturized, rechargeable battery operated, highly configurable and can be provided to stimulate one channel or multiple channels (*e.g.,* 2, 3, 4, 5, 6, 7, 8, 12, 16, *etc*.) channels independently, or synchronously, or a combination of both (*e.g.,* a subset of channels are synchronized while other channels are independent).

In various embodiments the portable stimulator is small and operated with a rechargeable battery so that it can be worn by a patient. In certain embodiments the portable stimulator is highly configurable. In certain embodiments the portable stimulator can be voice operated and/or operated using a app on a tablet, computer, or cell phone.

This device can be used to apply a stimulus to the spinal cord (cervical, thoracic, lumbar) to treat conditions of paralysis, respiratory arrest/failure/ventilator dependency, and the like.

In certain embodiments (*e.g*., as described below), the stimulator can have adjustments for stimulation primary frequency, amplitude, and pulse width in addition to carrier frequency, amplitude, and pulse width. The device can be manufactured in different numbers of channels (*e.g*., from 1 up to 16 or 32) with independent controls for each channel or controls to operate multiple channels in consort. In certain embodiments the number of channels controlled by a particular control set can be set by software and/or by hardware.

Currently there are no devices that addresses the primary indication(s) contemplated herein which include, but are not limited to respiratory, bladder, motor, and sexual dysfunction. There are devices that treat pain but the target organ for such stimulation is typically peripheral nerves for local muscles. In contrast, the portable stimulators contemplated herein are configured to targets the spinal cord or spinal nerve roots. While there are existing epidural spinal cord stimulators that target the spinal cord, however, these devices are for implantation and requires surgery. In contrast, the portable stimulators described herein is non-invasive, portable, and has latitude for adjustments in terms of stimulation parameters.

Additionally, it is noted that phrenic nerve/diaphragm pacemakers have been devised to address diaphragm paralysis that leads to impaired breathing. However, the natural respiratory drive is not addressed by such stimulators (in contrast to the methods and stimulators described herein) and the location of intervention is different from that described herein (*e.g*., phrenic nerve vs. spinal cord/nerve root stimulation). The stimulation modalities described herein recapitulate the afferent sensory feedback associated with respiration (CO₂, tidal volume, rate, etc.). This application activates the respiratory drive that is responsive to normal feedback to respiration by using a device that is non-invasive in nature.

TENS units are portable devices used to treat pain. However, there are no TENS devices that are optimized for the spinal cord with parameters that can be changed (intensity, frequency, carrier frequency) to the specific needs of the patient as determined by the physician. Furthermore, there are no devices that is portable and responsive to voice.

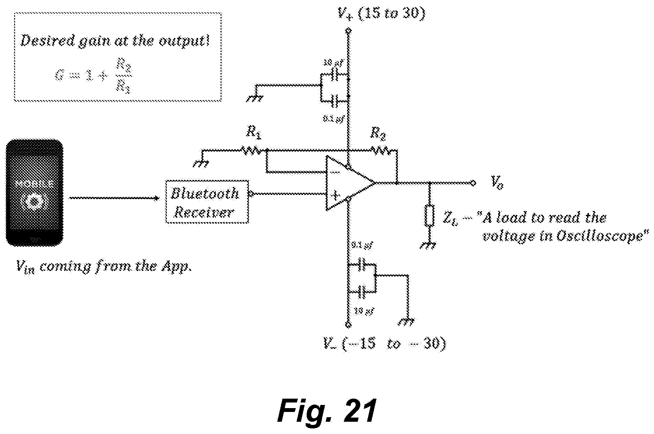

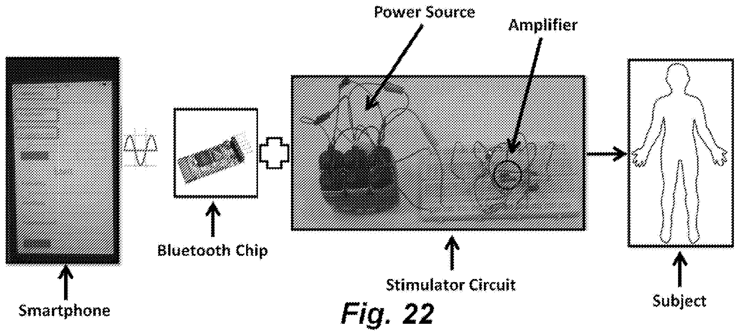

Examples of a portable stimulator are shown in Figures 21 and 22. These examples are illustrative and non-limiting. Using the teaching provided herein, numerous other portable stimulators suitable for transcutaneous stimulation (e.g., to stimulate and/or to maintain respiration) will be available to one of skill in the art.

In certain illustrative, but non-limiting embodiments, an electrical stimulator configured for transcutaneous electrical stimulation is provided where the said stimulator is portable, and battery powered; and the stimulator provides a signal for transcutaneous electrical stimulation superimposed on a high frequency carrier signal. In certain embodiments the stimulator provides user control over one or more parameters stimulation parameters, for example, stimulation frequency, and/or stimulation amplitude, and/or stimulation pulse width, and/or carrier frequency, and/or carrier frequency pulse width, and the like. In certain embodiments any one or more of these parameters are under the user's (patient's) control. In certain embodiments any one or more of these parameters are under the control of a health care provider.

In certain embodiments the stimulator is configured to provide a stimulation frequency ranging from about 0.5 Hz up to about 200 Hz. In certain embodiments the stimulator is configured to provide a stimulation amplitude ranging from about 1 mA up to to about 1000 mA. In certain embodiments the stimulator is configured to provide a stimulation pulse width ranging from about 0.1 msec up to about 100 msec. In certain embodiments the stimulator is configured to provide a carrier signal frequency ranging from about 1 kHz up to about 100 kHz. In certain embodiments the stimulator is configured to provide a carrier signal pulse width compatible with the above-described stimulation and carrier parameters.

In certain embodiments the stimulator is configured to receive signals from a heart rate monitor and/or respiration monitor and to adjust respiration parameters in response to changes in heart rate and/or respiration patterns. In certain embodiments the stimulator is configured to provide transcutaneous stimulation in a method according to any one of the transcutaneous stimulation methods described herein.

In certain embodiments the stimulator is controlled by one or more controls on the stimulator. In certain embodiments the stimulator is remotely controlled (*e.g*., from a remote site, *e.g*., in response to respiration, blood oxygen, heart rate signals transmitted to that site and/or in accordance with a medical plan. In certain embodiments the stimulator is controlled (*e.g*., one or more stimulation parameters are controlled) by an application on a computer, a smartphone, or a tablet. In certain embodiments the stimulator is voice controlled.

### Magnetic stimulators.

Magnetic nerve stimulators are well known to those of skill in the art. Stimulation is achieved by generating a rapidly changing magnetic field to induce a current at the nerve of interest. Effective nerve stimulation typically requires a current transient of about 10⁸ A/s. In certain embodiments this current is obtained by switching the current through an electronic switching component (*e.g.*, a thyristor or an insulated gate bipolar transistor (IGBT)).

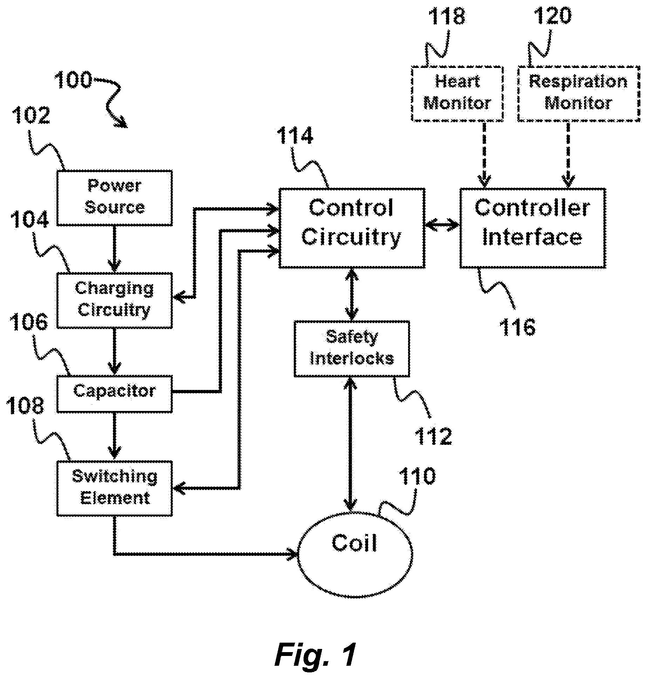

Figure 1 schematically shows one illustrative, but non-limiting embodiment of a magnetic stimulator. As shown therein, magnetic nerve stimulator **100** comprises two parts: a high current pulse generator producing discharge currents of, *e.g.,* 5,000 amps or more; and a stimulating coil **110** producing magnetic pulses (*e.g*., with field strengths up to 4, 6, 8, or even 10 tesla) and with a pulse duration typically ranging from about 100µs to 1ms or more, depending on the stimulator type. As illustrated in Figure 1, a voltage (power) source **102** (*e.g.,* a battery) charges a capacitor **106** via charging circuitry **104** under the control of control circuitry **114** (*e.g.,* a microprocessor) that accepts information such as the capacitor voltage, power set by the user, and various safety interlocks **112** within the equipment to ensure proper operation, and the capacitor is then connected to the coil via an electronic switching component **108** when the stimulus is to be applied. The control circuitry is operated via a controller interface **116** that can receive user input and/optionally signals from external monitors such as optional heart rate monitors **118** and/or optional or respiration monitors **120** and adjust stimulus parameters in response to variations/changes in those signals.

When activated, the discharge current flows through the coils inducing a magnetic flux. It is the rate of change of the magnetic field that causes the electrical current within tissue to be generated, and therefore a fast discharge time is important to stimulator efficiency.

As noted earlier the magnetic field is simply the means by which an electrical current is generated within the tissue, and that it is the electrical current, and not the magnetic field, that causes the depolarization of the cell membrane and thus the stimulation of the target nerve.

Since the magnetic field strength falls off with the square of the distance from the stimulating coil, the stimulus strength is at its highest close to the coil surface. The stimulation characteristics of the magnetic pulse, such as depth of penetration, strength and accuracy, depend on the rise time, peak electrical energy transferred to the coil and the spatial distribution of the field. The rise time and peak coil energy are governed by the electrical characteristics of the magnetic stimulator and stimulating coil, whereas the spatial distribution of the induced electric field depends on the coil geometry and the anatomy of the region of induced current flow.

The stimulating coils typically consist of one or more well-insulated copper windings, together with temperature sensors and safety switches.

In certain embodiments the use of single coils is contemplated. Single coils are effective in stimulating the human motor cortex and spinal nerve roots. To date, circular coils with a mean diameter of 80-100mm have remained the most widely used magnetic stimulation. In the case of circular coils the induced tissue current is near z on the central axis of the coil and increases to a maximum in a ring under the mean diameter of coil.

A notable improvement in coil design has been that of the double coil (also termed butterfly or figure eight coil). Double coils utilize two windings, normally placed side by side. Typically double coils range from very small flat coils to large contoured versions. The main advantage of double coils over circular coils is that the induced tissue current is at its maximum directly under the center where the two windings meet, giving a more accurately defined area of stimulation.

The stimulating pulse may be monophasic, biphasic or polyphasic. Each of these has its own properties and so may be useful in particular circumstances. For neurology, single pulse, monophasic systems are generally employed; for rapid rate stimulators, biphasic systems are used as energy must be recovered from each pulse in order to help fund the next. Polyphasic stimulators are believed to have a role in a number of therapeutic applications.

Descriptions of magnetic nerve stimulators can be found, *inter alia,* in U.S. patent publications US 2009/0108969 A1, US 2013/0131753 A1, US 2012/0101326 A1, IN U.S. Patent Nos: US 8,172,742, US 6,086,525, US 5,066,272, US 6,500,110, US 8,676,324, and the like. Magnetic stimulators are also commercially availed from a number of vendors, *e.g*., MAGVENTURE^{®}, MAGSTIM^{®}, and the like.

### Respiration stimulation/maintenance systems.

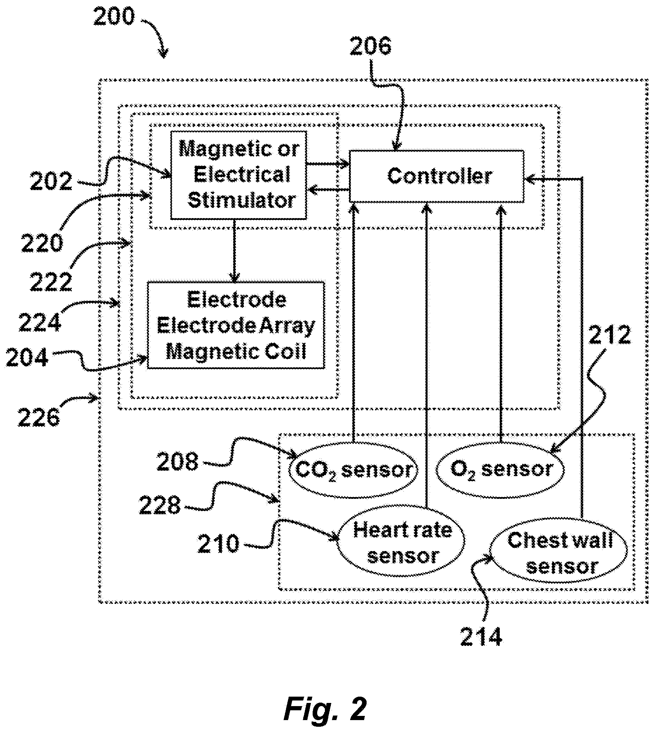

In certain embodiments, respiration stimulation/maintenance systems are contemplated. One illustrative, but non-limiting embodiment is shown in Figure 2. As shown therein the system **200** typically comprises an electrical or magnetic stimulator configured to induce epidural and/or transcutaneous electrical stimulation and/or magnetic stimulation. The electrical stimulation is delivered to a region of the cervical, and/or thoracic, and/or lumbar spine via a transcutaneous electrode, an epidural electrode (*e.g*., an epidural electrode array), or one or more magnetic coils **204.** In various embodiments a controller **206** regulates the stimulation parameters produced by the stimulator. In certain embodiments, the controller can be a unit or module that is separate from the stimulator, while in other embodiments in certain embodiments, the controller and stimulator can be integrated (as illustrated by dashed box **220).** In various embodiments the system typically also comprises one or more sensors. Illustrative, but non-limiting examples of sensors include an oxygen sensor **212,** a CO₂ sensor **208,** a chest wall expansion/movement sensor **214,** a heart rate sensor **210,** and the like. Typically, the output of the sensor(s) is coupled to the stimulator/controller so that the controller/stimulator adjust the stimulation pattern in response to the sensor output to provide a desired tidal volume and/or O₂ saturation and/or end tidal CO₂.

In certain embodiments the sensors are provided as integral components of the system. In other embodiments, the sensors are provided as instrumentation, *e.g*. in an acute care/intensive care unit, an emergency room, an operating room, *etc.*

In certain embodiments where a transcutaneous or magnetic stimulation is utilized the stimulator and associate coil/electrode can be external to the subject. Additionally, the other components of the system controller and various sensors can also be external.

In certain embodiments various components of the system are implantable into the subject (*e.g*., patient). Thus, for example, as indicated by dashed box **228** one or more of the sensors can be implantable sensors. In certain embodiments the electrode or coil **204** can be implanted while the stimulator and/or controller are external to the subject (as indicated by dashed box **222).** In certain embodiments the controller **206** and/or the stimulator **202** and the electrode or coil **204** are all implanted into the subject (as indicated by dashed box **224).** In certain embodiments the entire system is implanted into the subject (as indicated by dashed box **226).**

In certain embodiments the system comprises an implanted (*e.g*., surgically implanted), closed loop epidural stimulation device for spinal cord injured, stroke subjects, ALS patients with respiratory issues, and the like.

In certain embodiments the system comprises a temporary implanted device by percutaneous insertion of leads for ICU/acute care patients with acute respiratory failure to restore respiratory function or facilitate vent weaning. Such a system can provide a feedback mechanism assessing chest wall movement, O₂ saturation, end tidal CO₂ (all of which can be implemented in an acute care, *e.g.,* ICU bed setting) which adjusts the stimulation parameters.

In certain embodiments the system comprise a magnetic or transcutaneous electrical stimulation device for SIDS or ICU patients with decreased respiratory drive and can provide a feedback mechanism that monitors chest expansion, and/or and O₂ saturation , and/or CO₂ (pCO₂) and uses this information to adjust stimulation parameters.

Sensors for detecting a variety of physiological parameters including, but not limited to O₂ saturation, pCO₂, chest wall movement, heart rate and the like are well known to those of skill in the art. For example, external and implantable oximeters can routinely determine O₂ saturation. A typical pulse oximeter utilizes an electronic processor and a pair of small light-emitting diodes facing a photodiode through a translucent part of the subject's body, typically a fingertip or an earlobe. In one illustrative, but non-limiting embodiment, one LED is red, *e.g.,* with a wavelength of about 660 nm, and the other is infrared, *e.g.,* with a wavelength of about 940 nm. Absorption of light at these wavelengths differs significantly between blood loaded with oxygen and blood lacking oxygen. Oxygenated hemoglobin absorbs more infrared light and allows more red light to pass through. Deoxygenated hemoglobin allows more infrared light to pass through and absorbs more red light. The LEDs sequence through their cycle of one on, then the other, then both off, *e.g.,* about thirty times per second which allows the photodiode to respond to the red and infrared light separately and also adjust for the ambient light baseline. The amount of light that is transmitted is measured, and separate normalized signals are produced for each wavelength. These signals fluctuate in time because the amount of arterial blood that is present increases (literally pulses) with each heartbeat. By subtracting the minimum transmitted light from the peak transmitted light in each wavelength, the effects of other tissues are corrected for. The ratio of the red light measurement to the infrared light measurement is then calculated by the processor (which represents the ratio of oxygenated hemoglobin to deoxygenated hemoglobin), and this ratio is then converted to SpO₂ *e.g.,* by a processor via a lookup table based on the Beer-Lambert law. External oximeters are commercially available and are routinely available in acute care settings (*e.g.,* emergency room, ICU, operating rooms, *etc.*).

Implantable oximeters function in a similar manner. The implantable oximeter typically includes a light emitting diode, that transmits light into blood passing the sensor, and a phototransistor that senses the light after it has passed through the blood. Blood SO2 is then derived from a comparison of the intensity and frequency of the emitted light and the received light. Depending upon the implementation, an optical measurement window of the phototransistor of the sensor is either positioned within the blood stream (such as within one of the chambers of the heart) or is positioned near a blood vessel (*e.g*., subcutaneously). Implantable oximeters are known to those of skill in the art. See, for example, U.S. Patent No: 8,099,146 B1 and the references cited therein.

Capnography is the monitoring of the concentration or partial pressure of carbon dioxide (CO₂). A capnogram is a direct monitor of the inhaled and exhaled concentration or partial pressure of CO₂, and an indirect monitor of the CO₂ partial pressure in the arterial blood. Capnographs usually work on the principle that CO₂ absorbs infrared radiation. A beam of infrared light is passed across the gas sample to fall on a sensor. The presence of CO₂ in the gas leads to a reduction in the amount of light falling on the sensor, which changes the voltage in a circuit. The analysis is rapid and accurate. Capnographs are commercially available and routinely found in acute care settings (*e.g.,* ICU, emergency room, operating rooms, *etc.*)*.* One illustrative capnograph is the Micro-Capnograph produced by Linton Instrumentation, Inc.

In certain embodiments pCO₂ can be Measured using a transcutaneous monitor (*e.g*., available from Radiometer America, and the like).

PC0₂ can also be measured using an implantable CO₂ sensor (*see, e.g,* Čajlakovi ć*et al.* (2012), *Optochemical Sensor Systems for In-Vivo Continuous Monitoring of Blood Gases in Adipose Tissue and in Vital Organs,* pages 63-88 in *Chemical Sensors,* Wen Wang, ed., InTech.).

Sensors for measuring/monitoring chest wall expansion and/or movement are also well known to those of skill in the art. For example, rib cage movement can be measured with an inductance or strain gage band placed around the rib cage, *e.g.,* immediately below the axillae. In the case of an inductance band, chest expansion can be determined by changes in the inductance of the band induced by stretching of the band (*see. e.g.,* Drummond et al. (1996) Br. J. Anaesthesia, 77: 327-332). Similarly, in a strain gage band, changes in resistance/conductance of the strain gages produced by band expansion/contraction can readily be measured using methods known to those of skill in the art.

In another illustrative, but non-limiting embodiment, chest wall expansion and/or movement can be monitored by measuring thoracic impedance, *e.g*., as described by Drummond et al. (1996) Br. J. Anaesthesia, 77: 327-332.

In another illustrative, but non-limiting embodiment, chest wall expansion and/or movement can be monitored using a laser, *e.g.,* as described by Kondo et al. (1997) Eur. Respir. J. 10: 1865-1869.

In certain embodiments chest wall position/movement can be monitored using an accelerometer. In various embodiments the accelerometer can be attached to the surface of the body, while in other embodiments, the accelerometer can be implanted within the body. Implantable and surface accelerometers configured for the detection of chest wall motion are described, for example, in U.S. Patent Pub. No: 2013/0085404.

It will be noted that these particular systems and sensors are illustrative and non-limiting. Using the teachings provided herein, numerous other systems will be available to one of skill in the art.

### Self-Contained Respiratory Regulation Devices.

In certain embodiments self-contained, portable, respiratory regulation devices/systems are contemplated. In various embodiments the systems comprise a stimulator and an electrode for applying an electrical stimulus or a coil for applying a magnetic stimulus. The electrode and/or coil are configured for application of a stimulus to a region of the spine (*e.g.,* a cervical, and/or thoracic, and/or lumbar region) of a subject to control and/or to induce respiration.

In one illustrative but non-limiting system a control module is operably coupled to stimulator and instructs the stimulator module regarding the signal to be generated. For example, at any given time or period of time, the control module may instruct the signal generation module to generate an electrical signal having a specified pulse width, frequency, intensity (current or voltage), *etc.* The control module may be preprogrammed prior to use or receive instructions from a programmer (or another source). Thus, in certain embodiments the pulse generator/controller is configurable by software and the control parameters may be programmed/entered locally, or downloaded as appropriate/necessary from a remote site.

In certain embodiments the pulse generator/controller may include or be operably coupled to memory to store instructions for controlling the stimulation signal(s) and may contain a processor for controlling which instructions to send for signal generation and the timing of the instructions to be sent.

In some embodiments the respiration control devices can be pre-programmed with desired stimulation parameters. In some instances, some or all parameters of the electrode may be controllable by the subject, *e.g*., without supervision by a physician. In other instances, some or all parameters of the electrode may be automatically controllable by a programmer or controller comprising the device.

Devices for neuromodulation of respiration can have a variety of configurations. In some instances, the device may be configured as a belt or strap having at least one electrode or coil operably attached thereto. Alternatively, the device may be configured as an adhesive patch having at least one electrode or coil operably attached thereto. The power source and/or stimulator can be integrally formed with, connected to the electrode(s) or coil(s). As noted, in certain instances the device can include a controller (as described above) that is configured, for example, to electronically communicate (*e.g*., wirelessly) with an electronic device (*e.g.,* a PDA, cell phone, tablet, computer, *etc.*)*.*

The device can be part of an open- or closed-loop system. In an open-loop system, for example, a physician or subject may, at any time, adjust treatment parameters, such as pulse amplitude, pulse-width, pulse frequency, duty cycle, *etc.* In certain embodiments this open loop can be used in conjunction with ventilator device during weaning off the ventilator. In certain embodiments it can be coupled or built into ventilator control with values from the ventilator (tidal volume, PEEP, frequency) fed back into the stimulation control unit, processed and stimulation algorithm implemented for stimulation.

Alternatively, in a closed-loop system, treatment parameters (*e.g*., electrical signals) may be automatically adjusted in response to a sensed physiological parameter or a related symptom indicative of the extent of respiration. In a closed-loop feedback system, a sensor that senses a physiological parameter associated with respiration (*e.g*., that senses heart rate, tidal volume, *etc*.) can be utilized.

It should be appreciated that incorporating the therapy delivery device as part of a closed-loop system can include placing a therapy delivery device on or within a mammal at a nerve target, sensing a physiological parameter associated with respiratory function, and then activating the delivery device to apply a signal to adjust the respiration parameters in response to the sensor signal.

### Use of neuromodulatory agents.

In certain embodiments, the transcutaneous and/or epidural and/or magnetic stimulation methods described herein are used in conjunction with various pharmacological agents, particularly pharmacological agents that have neuromodulatory activity (*e.g*., are monoamergic). In certain embodiments, the use of various serotonergic, and/or dopaminergic, and/or noradrenergic, and/or GABAergic, and/or glycinergic drugs is contemplated. These agents can be used in conjunction with epidural stimulation and/or transcutaneous stimulation and/or magnetic stimulation as described above. This combined approach can help to put the spinal cord in an optimal physiological state for controlling respiration.

In certain embodiments, the drugs are administered systemically, while in other embodiments, the drugs are administered locally, *e.g.,* to particular regions of the spinal cord. Drugs that modulate the excitability of the spinal neuromotor networks include, but are not limited to combinations of noradrenergic, serotonergic, GABAergic, and glycinergic receptor agonists and antagonists.

Dosages of at least one drug or agent can be between about 0.001 mg/kg and about 10 mg/kg, between about 0.01 mg/kg and about 10 mg/kg, between about 0.01 mg/kg and about 1 mg/kg, between about 0.1 mg/kg and about 10 mg/kg, between about 5 mg/kg and about 10 mg/kg, between about 0.01 mg/kg and about 5 mg/kg, between about 0.001 mg/kg and about 5 mg/kg, or between about 0.05 mg/kg and about 10 mg/kg.

Drugs or agents can be delivery by injection (*e.g*., subcutaneously, intravenously, intramuscularly), orally, rectally, or inhaled.

Illustrative pharmacological agents include, but are not limited to, agonists and antagonists to one or more combinations of serotonergic: 5-HT1A, 5-HT2A, 5-HT3, and 5HT7 receptors; to noradrenergic alpha l and 2 receptors; and to dopaminergic D1 and D2 receptors (see, *e.g.,* Table 1).

**Table 1. Illustrative pharmacological agents.**

| Name | Target | Action | Route | Typical Dose (mg/Kg) | Typical Range (mg/kg) |
|---|---|---|---|---|---|
| Serotonergic receptor systems | | | | | |
| 8-OHDPAT | 5-HT1A7 | Agonist | S.C. | 0.05 | 0.045-0.3 |
| Way 100.635 | 5-HT1A | Antagonist | I.P. | 0.5 | 0.4-1.5 |
| Quipazine | 5-HT2A/C | Agonist | I.P. | 0.2 | 0.18-0.6 |
| Ketanserin | 5-HT2A/C | Antagonist | I.P. | 3 | 1.5-6.0 |
| SR 57227A | 5-HT3 | Agonist | I.P. | 1.5 | 1.3-1.7 |
| Ondanesetron | 5-HT3 | Antagonist | I.P. | 3 | 1.4-7.0 |
| SB269970 | 5-HT7 | Antagonist | I.P. | 7 | 2.0-10.0 |

| Noradrenergic receptor systems | | | | | |
|---|---|---|---|---|---|
| Methoxamine | Alpha1 | Agonist | I.P. | 2.5 | 1.5-4.5 |
| Prazosin | Alpha1 | Antagonist | I.P. | 3 | 1.8-3.0 |
| Clonidine | Alpha2 | Agonist | I.P. | 0.5 | 0.2-1.5 |
| Yohimbine | Alpha2 | Antagonist | I.P. | 0.4 | 0.3-0.6 |

| Dopaminergic receptor systems | | | | | |
|---|---|---|---|---|---|
| SKF-81297 | D1-like | Agonist | I.P. | 0.2 | 0.15-0.6 |
| SCH-23390 | D1-like | Antagonist | I.P. | 0.15 | 0.1-0.75 |
| Quinipirole | D2-like | Agonist | I.P. | 0.3 | 0.15-0.3 |
| Eticlopride | D2-like | Antagonist | I.P. | 1.8 | 0.9-1.8 |

The foregoing methods are intended to be illustrative and non-limiting. Using the teachings provided herein, other methods involving transcutaneous electrical stimulation and/or epidural electrical stimulation and/or magnetic stimulation and/or the use of neuromodulatory agents to improve, and/or regulating, and/or restore respiration in a subject with a respiratory deficiency will be available to one of skill in the art.

### EXAMPLES

The following examples are offered to illustrate, but not to limit the claimed invention.

### Example 1

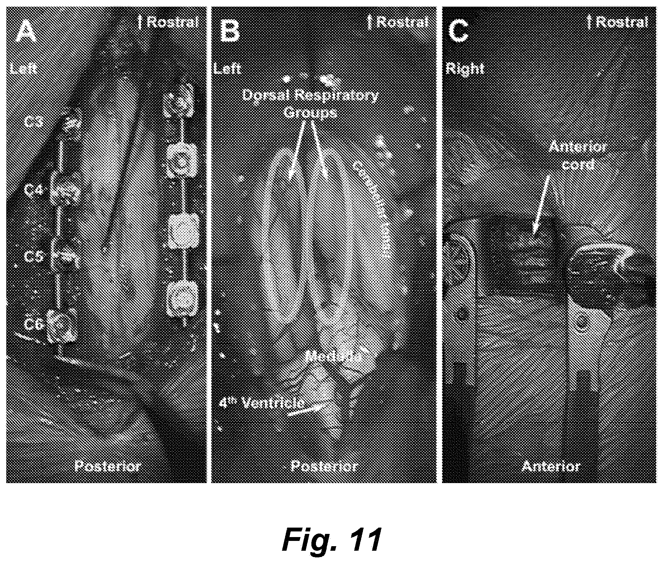

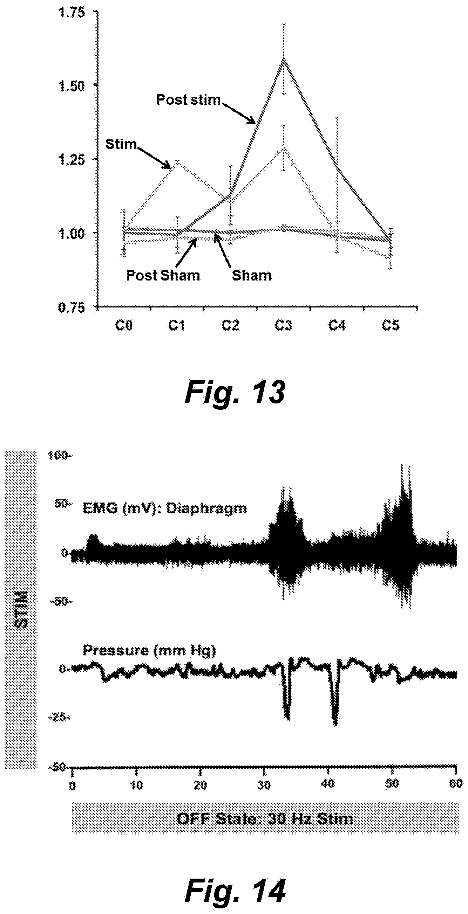

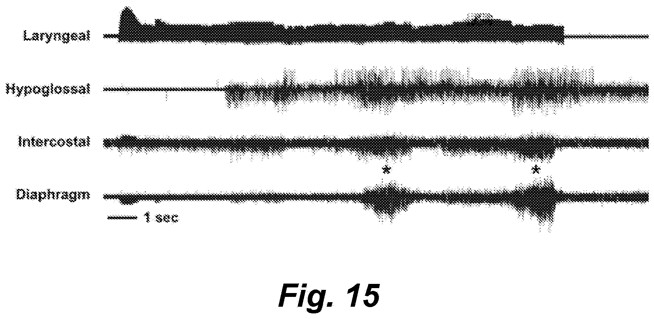

Figures 14 and 15 were obtained during a cervical spinal surgery in which the posterior elements (lamina and spinous process) of the spinal column was removed exposing the epidural space demonstrate the proof-of-concept in using epidural electrical stimulation to modulate respiration in humans (*see, e.g.,* Figure 11).

Monopolar ball-tipped electrodes were placed on top of the epidural space at different cervical locations (*see, e.g.,* Figure 11, panel C), frequency, and amplitude with pulse width of 450 us. Figure 14 shows that stimulation at a frequency of 30 Hz at C3/4 can induce respiration during deep anesthesia in humans. Electrical stimulation of the dorsal cord at C3/4 resulted in diaphragm EMG bursts and productive inhalation. This result indicates that the spinal cord stimulation can generate physiological respiratory/lung function.

Figure 15 shows that stimulation at a frequency of 30 Hz at C3/4 can induce coordinated respiration during off-state in humans. This observation indicates that accessible regions of the spinal cord can allow for coordinated respiratory/lung function following dysfunction or loss of connectivity with brainstem respiratory centers.

### Example 2

### Modulation of Respiratory Output by Cervical Epidural Stimulation in the Anesthetized Mouse.

### Abstract

Respiration is produced and controlled by well-characterized brainstem nuclei, but the contributions of spinal circuits to respiratory control and modulation remain under investigated. Many respiratory studies are conducted in *in vitro* preparations (*e.g.,* brainstem slice) obtained from neonatal rodents. While informative, these studies do not fully recapitulate the complex afferent and efferent neural circuits that are likely to be involved in eupnea (*i.e.,* quiet breathing). To begin to investigate spinal contributions to respiration, we electrically stimulated the cervical spinal cord during unassisted respiration in anesthetized, intact mice. Specifically, we used epidermal electrical stimulation at 20 Hz and varied current intensity to map changes in respiration. Stimulating at 1.5 mA at cervical level 3 (C3) consistently caused a significant increase in respiratory frequency compared to pre-stimulation baseline and when compared to sham stimulations. The increase in respiratory frequency persisted for several minutes after epidural stimulation ceased. There was no change in tidal volume. Sigh frequency also increased during epidural stimulation at C3. Neither the increase in respiratory frequency nor the increase in sighing was observed at other dorsal cervical levels. Increasing electrical stimulus intensity did not increase respiration frequency or increase the duration of increased respiration frequency, which suggests that the spinal circuits involved in the modulation of eupnea and sighing may be preferentially activated by specific endogenous inputs. These findings also suggest that the cervical spinal cord can play a role in respiratory modulation that affects both eupneic respiration and sigh production in intact, adult mice.

### Introduction

The mammalian respiratory system is controlled by a complex network of neurons interconnected among several nuclei located in the brainstem. The origin of the respiratory rhythm has been attributed to the pre-Bötzinger Complex (preBötC) located in the ventral medulla (Feldman et al. (1990) Am. J. Physiol. 259: R879-886; Onimaru et al. (2009) Resp. Physiol. Neurobiol., 168: 13-18; Smith et al. (1991) Science 254: 726-729) and the parafacial respiratory group (pFRG) located in the rostro-ventrolateral medulla (Duffin (2004) Exp. Physiol., 89: 517-529; Onimaru et al. (2006) J. Neurophysiol., 96: 55- 61). Pontine nuclei also seem important for generating the eupneic rhythm (Lindsey et al. (2012) Compr. Physiol., 2: 1619-1670; St. John and Paton (2004) Respir. Physiol. Neurobiol. 143: 321-332). The rhythm generating elements within the pons and medulla are intimately interconnected and embedded within neuron groups that shape the pattern of the respiratory output. Thus, within this larger respiratory central pattern generator, there are individual rhythm generating elements that come into play in a variety of different circumstances. Among the key structures for respiratory control are the ventral respiratory column (VRC), pontine respiratory group (PRG) and dorsal respiratory group (DRG) (Lindsey et al. (2012) Compr. Physiol., 2: 1619-1670). This network of neurons in turn activates the motor neurons of respiratory muscles in a highly choreographed sequence, which leads to remarkably stable and well-coordinated inspiratory and expiratory flow during activities as diverse as quiet eupnea, speech and vigorous exercise (Bartlett and Leiter JC (2011) Compr. Physiol. 2: 1387-1415). The respiratory central pattern generator (CPG) may overlap and coordinate with other CPGs so that the respiratory pattern may be involved in or encompassed within a variety of other activities, such as swallowing, coughing and sighing.

Speculation about the existence of spinal respiratory neurons with rhythmic properties in the spinal cord was raised when inspiratory unit activities were recorded in C1-C2 segments of transected cats (Aoki et al. (1980) Brain Res. 202: 51-63), and it believed that there are inspiratory neurons found in the upper cervical cord (Duffin and Hoskin (1987) Brain Res. 435: 351-354; Hoskin and Duffin (1987) Exp. Neurol., 95: 126-141; Hoskin and Duffin (1987) Exp. Neurol., 98: 404-417; Lipski and Duffin (1986) Exp. Brain Res. 61: 625-637). A study using an *in vitro* preparation of the neonatal mouse brainstem-spinal cord has shown that spontaneous rhythmic respiratory burst activity can be generated in the cervical ventral roots of C1/C2 and C4 following transection of the spinal cord above C1 (Kobayashi et al. (2010) J. Physiol. Sci. 60: 303-307).

To date, studies that have identified cervical spinal cord involvement in respiratory function have used *in vitro* testing (Aoki et al. (1980) Brain Res. 202: 51-63; Dubayle and Viala (1996) Neuroreport 7: 1175-1180; Duffin and Hoskin (1987) Brain Res. 435: 351-354; Kobayashi et al. (2010) J. Physiol. Sci. 60: 303-307). To gain a better understanding of the role of the upper cervical spinal cord in respiration, we examined the effect of electrical stimulation on respiratory activity in intact anesthetized mice. We applied direct, constant current stimulation to the epidural surface of the exposed spinal cord from the most caudal region of the medulla (C0) through C1 to C5 and recorded the respiratory output. The aim of our study was (1) to identify a stimulation intensity required to evoke a respiratory response and (2) to determine if there were an optimal cervical regions to stimulate in order to increase respiratory activity in healthy adult mice. Our results showed that epidural stimulation at the C3 level of the spinal cord with 1.5 mA increased the respiratory rate and the sigh frequency, but did not change the tidal volume in anesthetized mice. Our findings show that respiratory function, possibly involving two respiratory related CPGs - eupnea and sighing, can be altered by stimulation of the cervical spinal cord if the appropriate stimulation intensity and location are used.

### Methods

### Animals

Mixed gender 5-month-old C57BL/6 mice were used in this study. All mice were kept and tested according to protocols approved by Animal Research Committee (ARC) at University of California, Los Angeles. All procedures were conducted on mice anesthetized by isoflurane. All mice were subjected to sham and monopolar electric epidural stimulations of the spinal cord.

### Laminectomy

To perform the laminectomy, the animal was placed under anesthesia and spread prone on a surgical pad. The laminae from the C1 to C6 vertebral levels were gently removed to expose the spinal cord with intact dura matter. Gauze pads soaked in mineral oil were placed on top of the spinal cord to keep it moist, and the dorsal skin on either side of the wound was carefully clipped together with surgical clips to keep the gauze in place. The animal was then positioned for a tracheostomy and intubation.

### Tracheostomy and intubation

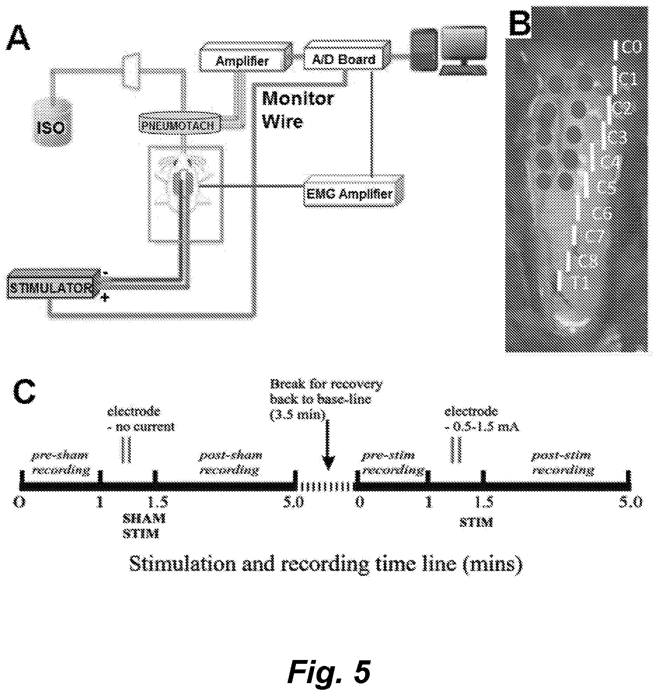

Respiratory flows were measured in anesthetized and tracheostomized mice. The tracheostomy and intubation methods were adapted from the protocol described by Moldestad et al. (2009) J. Neurosci. Meth. 176: 57-62. Briefly, a 50~60 mm intravenous catheter (Venflon Pro. BD, 18-20 GA, BD, NJ, USA) was inserted gently between two tracheal rings, and its position was secured by super glue. The animal was observed for 5 min to detect any possible respiratory problems before the super glue completely solidified. Finally, the animal was connected to a pneumotachograph and moved into position for epidural stimulation (see Figure 5, panel A). Epidural electrical stimulation protocol Figure 5, panel B, shows the dorsal spinal surface after the laminectomy, and Figure 5, panel C, illustrates the epidural stimulation protocol at each cervical level. This protocol was used in all the studies reported here. Pre-sham-baseline respiratory activity was recorded for 2 min, followed by sham epidural stimulation. To conduct the sham stimulation, the electrodes were gently pressed onto the epidural surface of the spinal cord for 30 s with no current applied. The post-sham-baseline was recorded immediately after for 3 min. The pre- stimulation baseline was then recorded for a further 3 min followed by a 30s electrical stimulation. Finally, a 5 min post-stimulation baseline was recorded. The same sequence of sham and active epidural stimulation was repeated on the next cervical level in a randomized sequence (C0-C5).

### Testing for electrical stimulation intensity and location in cervical spinal cord to evoke a respiratory response.

We first carried out a study to determine if there were suitable intensities and cervical spinal cord levels to evoke a respiratory response in the anesthetized mouse. In this study, 13 mice were used. We tested the brainstem (C0) and C1 to C5 cervical levels with stimulus strengths of 0.3 mA, 0.9-1.0 mA and 1.5 mA using a constant current stimulator. For this study, all the stimulations were performed along the dorsal medial-lateral surface of the spinal cord, and all spinal cord levels were tested bilaterally in a randomized manner. A constant stimulation frequency of 20 Hz and monophasic electrical stimulation, 1% duty cycle and square wave pulse pattern were used in this study. The ground was placed on the spinal cord 1 mm away from the stimulation electrode for these monopolar stimulations.

### Cervical C3 stimulation to examine changes in respiratory response

After the optimal stimulation intensity and cervical level were determined (see results section), a separate study was conducted to examine whether stimulation at each cervical level was sufficient to evoke a respiratory response. A total of 24 mice were used in this study. A laminectomy from C1 to C6 was performed as described above, and all animals were tested using the epidural stimulation protocol as described previously.

### Physiological recordings

For all the studies, diaphragmatic electromyographic (EMG) recordings were carried out. A 3-cm incision was made at the base of the rib cage to expose the abdominal surface of the diaphragm. Monopolar electrodes were inserted medially into the diaphragm, and EMG muscle activity was recorded. The EMG recording wires were connected to an amplifier, and the EMG signal was band-pass filtered between 30 and 3,000 Hz and digitized at 10 KHz. An amplifier (3000 AC/DC Differential Amplifier, AM system, Carlsborg, WA), an A/D board (DT1890, Data Translation Inc., Marlboro, MA) and a data analysis software system (DataView, St. Andrew University) were used to record the EMG signals for subsequent analysis.

### Data collection

The respiratory patterns as well as vital physiological variables (heart rate, peripheral oxygen saturation, respiration rate, and body temperature) of tested animals were monitored throughout the whole test using the PhysioSuiteTM system (Kent Scientific). The respiratory flow was monitored through a pneumotachograph and pressure transducer (Biopac Systems, Inc., Goleta, CA) and recorded on a computer using DataView (University of St Andrews). The digitization rates of all the channels were 10 KHz.

### Data analysis.

For each round of sham and stimulation recording, the data used for analysis were as follows: 1 min pre-sham, 30 sec sham, 3 min post-sham, 1 min pre-stimulation, 30 sec stimulation and 3 min post-stimulation. The respiratory frequency and peak-to-peak amplitude (tidal volume) were extracted. The extracted data were de-noised with a running average filter (time constants = 250 ms), normalized (rectified and smoothed with a Gaussian filter) and visualized through MATLAB (MathWorks, Natick, MA). The analyzed data were presented as a ratio comparing sham to pre-sham, post-sham to pre- sham, stimulation to pre-stimulation, and post-stimulation to pre-stimulation. These data were also used to analyze the occurrence of sighs. Sighs have a characteristic flow morphology and are followed by a brief expiratory pause. Sighs were typically about twice the volume of a normal breath (3). Sigh data were presented as the absolute number of sighs and absolute frequency. Patterns of sigh and eupnea frequency response for each mouse tested were compared to each other to examine if there was a correlation between the sigh response and the eupneic response to epidural stimulation.

The response to epidural stimulation at any spinal level differed among animals. In order to objectively categorize the different patterns of responses, we employed cluster analysis, which can be used to systematically and hierarchically categorize data based on the mathematical similarity of the responses. We performed the hierarchical cluster (Hclust) analysis using R software (www.r-project.org) where each trace of the ratio of respiratory activity during stimulation was compared to baseline respiratory activity over time. Hclust uses a distance matrix based on dissimilarity (calculated as the square root of sum of squares of differences in particular attributes) and estimates the Euclidean distance between different data sets (respiratory frequency responses to epidural stimulation in our case). The greater the distance between two patterns of response, the greater the difference in the shape of each trace of respiratory frequency ratios.

For our analysis, we analyzed each point on the ratio of respiratory frequency at 0.25 secs intervals to estimate the differences among frequency responses among animals. If the calculated distance was smaller than 7.5 hierarchical units, the recording was considered to be in the same cluster. If the distance was larger than 7.5 hierarchical units, the recording was placed in a separate cluster. The distance cut-off of 7.5 was validated post-hoc by checking the standard error of the mean within clusters as well as through checking the correlation within and between the clusters. The Kendall Rank Coefficient test and Kendall's Tau-b were used for correlation validation. Both tests are used to test whether the variables within each cluster are truly different. Different cut-off values (2.5, 5, 7.5, 10 and 12.5) were tested with these two tests, and 7.5 was the minimum distance for the variables to achieve correlation validation.

### Statistics.

A one-way, repeated measures ANOVA was used for all data analyses. If the ANOVA indicated that significant differences existed among treatments, pre-planned paired comparisons were made using p-values adjusted by the Bonferroni method. In all cases, p < 0.05 was considered statistically significant.

### Results

### Optimization of current intensities

Systematic stimulation of the brainstem (C0) and C1 to C5 cervical levels using different current intensity was carried out to identify the optimal stimulation intensity and cervical level required to elicit a respiratory response. At C0 and C1, there were no changes in respiratory frequency when 0.3 and 1.0 mA stimulation were used (Figure 6). Stimulation at 1.5 mA induced cardiac arrest, and therefore, this stimulation intensity was not continued at these cervical levels. Electrical stimulation at C2 and C4 at 0.3, 0.9-1.0 and 1.5 mA did not significantly increase respiration frequency (Figure 6). At the C3 level, 0.3 and 1.0 mA stimulation did not affect the respiratory frequency. However, 1.5 mA stimulation at C3 significantly increased the respiratory frequency during the period of stimulation (p = 0.0387). The increase in respiratory frequency also continued during the post-stimulation stages of recording even though stimulation had ceased (Figure 6, see *; p = 0.0017). Only 1.5 mA current intensity was tested at C5 as the location was close to a major blood vessel that impeded epidural stimulation. No respiratory change was observed at C5 using the 1.5 mA stimulation strength (Figure 6).

### Stimulation of C3 with 1.5 mA increases respiratory frequency but not the tidal volume

We determined that 1.5 mA at C3 was the optimal stimulation and location to elicit a change in respiratory frequency. Subsequently, we examined in-depth the frequency response elicited when C3 was stimulated with an electrical current of 1.5 mA. Our results show that there was a significant increase (p < 0.0001) in the ratio of stimulated respiratory frequency compared to the sham stimulation (Figure 7, panel A). The increase in respiratory frequency was seen during stimulation (see black arrow) and persisted in the post-stimulation phase (see red arrow). We only recorded for 3 min post-stimulation, and the duration of the facilitation of respiratory frequency may have persisted longer in some mice. We observed no significant change in the tidal volume of respiration when C3 was stimulated compared to the sham stimulation (Figure 7, panel B). Our results indicate that cervical epidural stimulation can increase the number of breaths taken, but not the depth of each breath.

### Hierarchical cluster analysis.

Using Hclust analysis we showed that the responses among animals could be grouped into different hierarchical clusters of respiratory responses when sham or epidural stimulation with 1.5 mA at C3 was carried out (Figure 8, panels A, B). Twenty-three out of 24 of the sham stimulation responses belonged to the same cluster (Figure 8, panel Ci). Only one of the 24 sham stimulation sequences fell into a different hierarchical cluster (Figure 8, panel Cii), indicating that during sham stimulation, respiration was unchanging and similar across virtually all sham stimulation sequences in all of the animals. When epidural stimulation was used, six hierarchical clusters were identified (Figure 8, panels D). The clusters differed in terms of the timing of the increase in respiratory frequency, and the persistence of the increase in respiratory frequency once epidural stimulation ceased. Clusters 1, 3, 4 and 6 contained 16 of the 24 animals studied (Figure 8, panel D i, iii, iv and v), and these clusters shared two features: the respiratory rate rose during epidural stimulation and the respiratory rate remained elevated at some level above baseline after epidural stimulation had been turned off. These four clusters differed only in the variability or level of the changes in respiratory frequency. Cluster 2 contained 6 of the 24 animals studied (Figure 4Dii), and in this cluster there was an initial increase in respiratory frequency that was not sustained after stimulation ceased. Cluster 5 contained 2 animals (Figure 4Dv) in which epidural stimulation did not alter respiratory frequency, but respiratory frequency rose and was sustained after epidural stimulation ceased. The final cluster contained only 1 animal (Figure 8, panel D vi) in which the respiratory frequency rose during epidural stimulation, and respiratory frequency was quite variable in the period following epidural stimulation. Even in clusters 2 and 5, all of the animals had some increase in respiratory frequency at some point during or immediately after epidural stimulation. In contrast, 23 of the 24 animals in the sham stimulation group had no discernible change in respiratory frequency.

### Stimulation with 1.5 mA at C3 increased the number of sighs

The number of sighs also increased significantly when mice received epidural stimulation of 1.5 mA at C3 compared to sham stimulation (Figure 5; see *, p = 0.0011). There was an increase in the number of sighs during stimulation compared to the pre-stimulation baseline (p = 0.002), and also during the 3 mins post-stimulation compared to the pre- stimulation baseline (p < 0.0001). Therefore, the number of sighs was elevated for up to 3 minutes after stimulation before returning to pre-stim baseline levels, but sighing diminished in many animals within 3 minutes after epidural stimulation stopped. These results differed from the eupneic respiratory frequency response where respiratory frequency was elevated for a more substantial part of the post-stimulation period and showed little evidence of abating in 18 of the 24 animals studied (clusters 1, 2, 4, 5 and 6).

### No correlation between patterns of sigh and eupnea frequency when stimulated with 1.5 mA at C3.

We assessed whether there was any correlation between the pattern of sigh and eupnea frequency responses after stimulation with 1.5 mA at C3. When each animal was stimulated, the pattern of sighing fell into one of four categories (much like the eupneic clusters). We simplified the categories of sigh and eupneic responses such that the categories included (1) no change during stimulation and no change after stimulation (only sighing clusters fulfilled these criteria), (2) no change during stimulation, but increased respiratory frequency post-stimulation (e.g., eupnea cluster, 5) (3) increased during stimulation, but without a sustained post-stimulation increase in respiratory frequency (e.g., cluster 3) and (4) increased during stimulation and sustained for some part of the post-stimulation period (*e.g.,* all the remaining eupnea clusters). We compared the sigh response pattern and the eupnea response patterns of each mouse during and after epidural stimulation and found no correlation between the pattern of frequency change of eupnea and the pattern of change of sigh frequency (Figure 10). In only 8 of 24 animals were the patterns of sighing and eupneic responses similar.

### Discussion.

The brainstem has been recognized as the main site for the generation of rhythmic respiratory drive based on a long history of studies in intact, anesthetized and decerebrate animals (Lindsey et al. (2012) Compr. Physiol., 2: 1619-1670; St. John (1990) J. Appl. Physiol., 68: 1305-1315; St. John and Paton (2004) Respir. Physiol. Neurobiol. 143: 321-332) and more recently on experiments using neonatal *in vitro* preparations (Feldman and Del Negro (2006) Nat. Rev. Neurosci. 7: 232-242; Onimaru et al. (2006) J. Neurophysiol., 96: 55- 61). The respiratory neurons are distributed in a number of different nuclei in the brainstem throughout the dorsal and ventral regions of the medulla and pons, and neurons within each nucleus make a unique and time dependent contribution to the generation of the pattern of respiratory activity. While not yet definitive, the cervical spinal cord may also contribute to respiratory rhythm generation, as spontaneous rhythmic breathing occurs in cervical spinalized dogs (Coglianese et al. (1997) Respir. Physiol. Neurobiol. 29: 247-254) and cats (Aoki et al. (1980) Brain Res. 202: 51-63). Further, *in vitro* studies have demonstrated the presence of inspiratory and pre-inspiratory neurons at the level of upper cervical spinal cord (Douse et al. (1992) Exp. Brain Res. 90: 153-162; Jones et al. (2012) Respir. Physiol. Neurobiol. 180: 305-310; Lipski and Duffin (1986) Exp. Brain Res. 61: 625-637; Oku et al. (2008) Neuroreport, 19: 1739-547 1743). However, *in vitro* studies, in artificial and reduced preparations do not fully recapitulate the physiological respiratory circuit for eupnea. The neural circuit generating and shaping eupnea is a large and complex network of interacting neurons that integrate multitude of afferent inputs and endogenously generated neural activity among multiple pontine, medullary and possibly spinal cervical locations. Therefore, we used epidural stimulation of the dorsal surface of the cervical spinal cord to assess the contribution of different cervical segments to respiratory neurogenesis in intact, anesthetized mice.

To our knowledge, this is the first study that examined respiratory function related to the cervical spinal cord in a whole mouse. Previous studies have used *in vitro* slice preparations or whole spinal cord/brain stem explants (Jones et al. (2012) Respir. Physiol. Neurobiol. 180: 305-310; Onimaru et al. (2006) J. Neurophysiol., 96: 55-61). By utilizing epidural stimulation in the cervical spinal cord, we found that 1.5 mA stimulation at C3 evoked a significant respiratory change; 1.5 mA stimulation at C3 increased the respiratory frequency during epidural stimulation, and in many animals, respiratory frequency remained elevated after epidural stimulation ceased. These respiratory frequency responses were only seen when epidural stimulation was delivered at C3 and not at levels C0 through C2 or C4 and C5. In contrast, tidal volume did not change during or after epidural stimulation at C3 or any other cervical level tested. The number of sighs also increased during epidural stimulation at C3, and the increase in sigh frequency also persisted after epidural stimulation stopped, but the facilitation of post-stimulation sighing was less persistent than the post-stimulation facilitation of the eupneic rhythm. Finally, there were differences among the responses of individual animals, and there appeared to be a clustering of respiratory frequency responses that largely reflected the timing and durability of the change in respiratory frequency during and after epidural stimulation.

We discovered that 1.5 mA, 20 Hz epidural stimulation delivered at the level of C3 significantly increased the respiratory frequency, but not the tidal volume. The location of this responsive center at C3 appears to be caudal to the C2 segment that demarcated the caudal border of high cervical respiratory group as defined by optical signals from a brainstem-spinal cord preparation (Oku et al. (2008) Neuroreport, 19: 1739-547 1743). This may indicate that there are heretofore unrecognized elements of the respiratory CPG caudal to C2, or there may be unique electrophysiological properties at C3 that allow current administered at C3 to influence more rostral respiratory CPG function without the C3 region possessing CPG activity of its own. If the response to stimulation at C3 were derived from current spread to C2 and more rostral elements, we might have expected direct stimulation of C1 and C2 to have a larger effect on respiratory frequency, and we found no effect of epidural stimulation at these spinal levels at all or the stimulation was fatal to the mouse.

The spinal cord is segmentally organized, and there is a tendency to focus on the repetitive, similarity of each spinal segment. The unique susceptibility of the C3 level to modulation by epidural stimulation suggests that there are specific, non-segmentally repeated elements at C3 that contribute to or are part of the neural systems that generate eupnea and sighing.

One unique feature of the C3 locus is that it is a site of integration of sensory information particularly related to the spinal trigeminal nucleus (STN) (2, 24). At the level of the medulla, STN, hypoglossal nucleus, and pre-Bötzinger complex are all reasonably close together and possess well-established reciprocal connections (Feldman and Del Negro (2006) Nat. Rev. Neurosci. 7: 232-242). It is possible that stimulation of C3 activates the afferent sensory fibers of STN which then influence the respiratory rhythm. There may be other tracts that are activated by C3 stimulation (e.g., medial longitudinal fasciculus) that integrate and coordinate the activity of brainstem nuclei. Although it is interesting that any rostrally projecting axons of passage might have been affected by epidural stimulation, there was no increase in respiratory activity when the C1 and C2 levels were stimulated, which argues against simple activation of rostrally projecting axons of passage. Thus, elucidating the mechanism(s) of respiratory responses to epidural stimulation at C3 and determining what unique features of C3 generate this response will require further studies.

The mechanism of modulation of the cervical respiratory network is likely through the interneuronal cervical respiratory circuit rather than direct phrenic motor neuron activation as there was no tonic respiratory muscle contraction during eupnea during epidural stimulation, but rather an increase in respiratory frequency. We performed a cluster analysis of the patterns of respiratory sigh activity. The cluster analysis of pre-, intra-, and post-stimulation conditions and sigh data also support the proposed mechanism of accessing the cervical interneuronal network rather than direct phrenic motor neuron activation as the frequency of sighing was also modulated, and sighs were present rather than unaffected or absent. The increase in sigh frequency during C3 stimulation (Figure 8) along with clustering of respiratory patterns (Figure 9) support the idea that the C3-brainstem respiratory circuit is connected and integrated with the ponto- medullary respiratory CPG since epidural stimulation affected the frequency of eupnea and the frequency of sighs, both of which have the CPGs within the brainstem. Moreover, the pattern of responses to epidural stimulation of eupnea and sighing were not correlated in individual animals. It seems unlikely that complete and separate CPGs for sighing and eupnea would be represented in the cervical spine, and so the dual effect of epidural stimulation strongly favors the hypothesis that cervical spinal neurons are part of an extended set of respiratory CPGs throughout the brainstem and upper cervical spine that support both sighing and eupnea. That two forms of respiratory activity were modified by epidural stimulation at C3 also makes it unlikely that the respiratory responses arose from a direct effect on motor neurons, which would have been expected to modify the duration or intensity of inspiratory and/or expiratory muscle activity, which we did not see.

Sighing has been associated with the retrotrapezoid nucleus/parafacial respiratory group (RTN/pFRG) and projects to preBötC (Onimaru et al. (2009) Resp. Physiol. Neurobiol., 168: 13-18), while eupnea is related to a complex interaction of brainstem respiratory nuclei (Lindsey et al. (2012) Compr. Physiol., 2: 1619-1670; St. John (1988) Prog. Neurobiol. 56: 97-117). Stimulation of a topographically distinct location in the cervical spine with associated alterations in both eupneic and sighing frequency and respiratory pattern suggests a reciprocally connected circuit between the respiratory centers for sighing and eupnea and the spinal cord and brainstem. The data gathered here are consistent with a hierarchical model of independent CPGs related to respiratory function in which the C3 region is a node or nexus of access to the respiratory circuit.

Interestingly, we discovered that there is an impact of stimulation on respiratory frequency that persists after removal of the stimulation. Such an observation supports the theory that spinal cord stimulation activates the cervical spinal motor circuit and lowers the threshold of activation or resting membrane potential for motor tasks with persistent and residual effects (Lu et al. (2016) Neurorehab. Neural Repair., 1545968316644344). One might imagine that the increased respiratory frequency occurs by facilitation or disinhibition or both. In favor of facilitation, it is noteworthy that a sustained increase in respiratory frequency, termed long term facilitation, follows intermittent hypoxia (8), and this long term facilitation seemed to depend on serotonergically induced plasticity. The epidural stimulation is clearly different from intermittent hypoxia, but epidural stimulation seemed to induce a similarly sustained increase in respiratory frequency in many of the animals, and serotonin has been used to enhance respiratory and motor function after spinal cord injury through a mechanism of increased spinal excitability and state permissive for motor activation (Choi et al. 92005) J. Neurosci. 25: 4550-4559; Courtine et al. (2009) Nat. Neurosci. 12: 1333-1342; Fong et al. (2005) J. Neurosci. 25: 11738-11747).

Disinhibition is also a possible mechanism of increasing the frequency of eupnea and gasping, and there are many GABAergic interneurons at each segmental level of the spine, which may have been modulated by epidural stimulation so as to allow a sustained increase in respiratory frequency. Moreover, there are examples of increased respiratory activity following spinal injury that have been attributed to disinhibition of these spinal interneurons (Lane et al. (2009) Respir. Physiol. Neurobiol., 169: 123-132). Defining the roles of facilitation and disinhibition (or both) in the response to epidural stimulation will also require further studies.

### Summary.

We found that epidural stimulation of the dorsal spinal cord at the C3 level increased the frequency of eupnea and sighing. Stimulation at no other cervical level from the caudal medulla to C5 had any effect on either eupnea or sighing. For both eupnea and sighing, the increase in frequency persisted after epidural stimulation ceased in many animals. The duration of post-stimulation frequency facilitation was greater for eupnea than sighing. These findings suggest that spinal elements at the C3 level make a unique and heretofore unappreciated contribution to respiratory frequency generation for both eupnea and sighing, probably through an interaction with or participation in the CPGs for eupnea and sighing. The mechanisms of the responses to epidural stimulation merit further study to elucidate the therapeutic potential of cervical epidural stimulation in individuals with spinal cord injuries.

### Example 3

### Intraoperative Stimulation of Humans

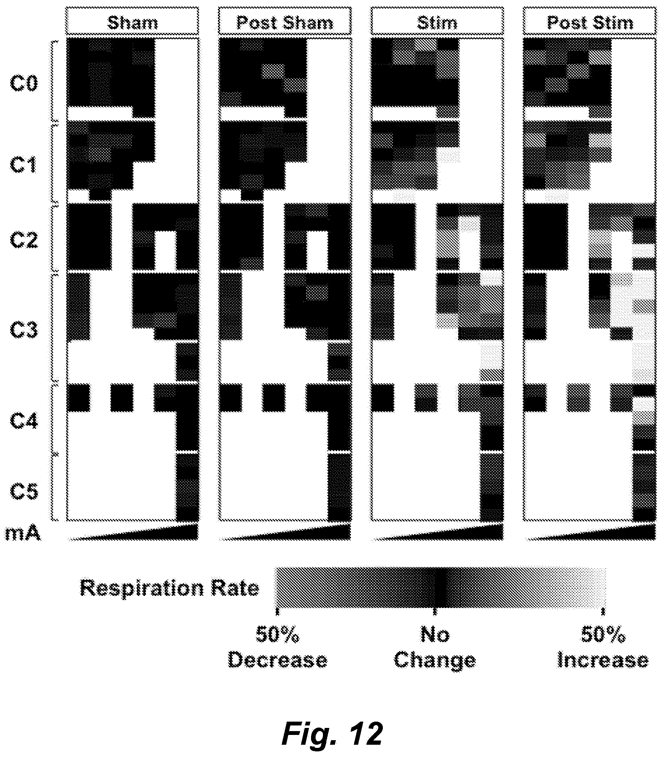

Figure 12 shows an overview of spinal respiratory stimulation. Anesthetized mice were monitored by pneumotach and EMG to monitor the respiratory rate. The heat map color code shown in Figure 12 reflects an increase (yellow) or decrease (blue) in respiratory rate. Measures are normalized to a 2-3 minute baseline. The mA scale reflects stimuli of increasing intensity from 0.3 to 1.5 mA. In the sham and post-sham conditions, very little change in respiration was observed. During stimulation, however, we observed substantial increases in respiratory rate when stimulating between 1 and 1.5 mA, notably at cervical levels, C2, C3 and C4. Post-stimulation, we saw continued modulation that persisted for 3 minutes. These results suggest that intermittent stimulation may be beneficial and stimulation duty cycle reduction s may be possible.

Figure 13 shows a summary of cervical respiratory stimulation in a mouse subjected to 30 Hz stimulation. Here the values for 1, 1.2, and 1.5 mA stimulation experiments are averaged. Prior to stimulation, in the sham and post-sham periods, very little change in respiration rate was seen. Substantial increases in respiratory rate were seen in stim and post-stim conditions, most notable at C3. (n=24).

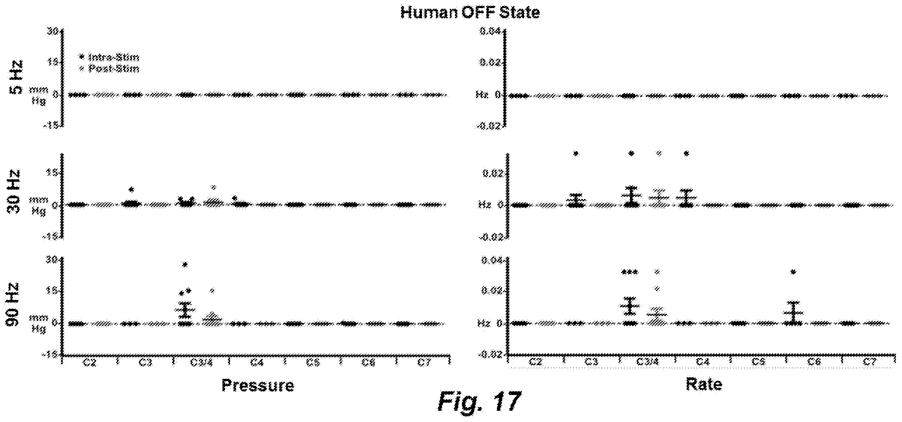

Figure 14 shows that stimulation at a frequency of 30 Hz at C3/4 can induce respiration during deep anesthesia in humans. Electrical stimulation of the dorsal cord at C3/4 resulted in diaphragm EMG bursts and productive inhalation. As in Figure 17 (described below), this result indicates that the spinal cord stimulation can generate physiological respiratory/lung function.

Figure 15 shows that stimulation at a frequency of 30 Hz at C3/4 can induce coordinated respiration during off-state in humans. This important experiment demonstrates that coordinated respiration can occur when stimulating the spinal cord. Note laryngeal and hypoglossal tone increasing prior to two breaths (*). This observation indicates that accessible regions of the spinal cord can allow for coordinated respiratory/lung function following dysfunction or loss of connectivity with brainstem respiratory centers.

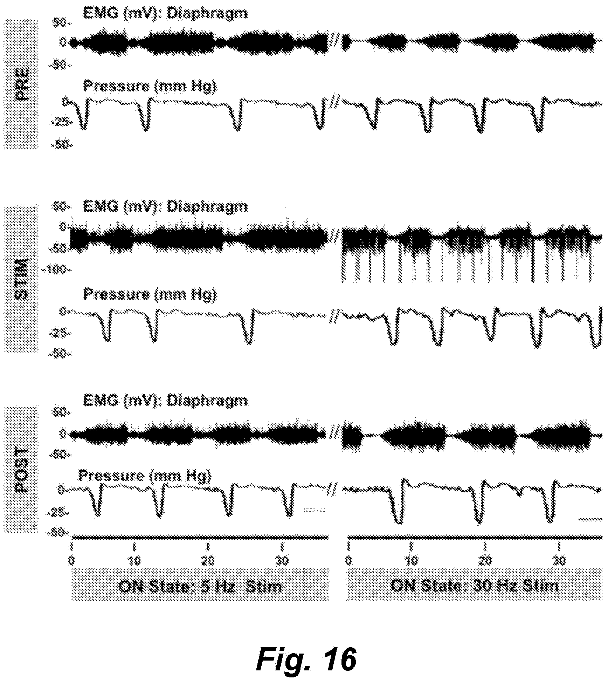

Figure 16 shows a representative respiratory response to stimulation at a frequency of 30Hz at C3/4 in humans. Diaphragm EMG and ventilator pressure were monitored under light sedation with spontaneous breathing (ON State). Top PRE condition shows no response at 5 Hz or 30 Hz. During STIM, 5Hz has no response, while 30Hz increased the rate. Following stimulation, POST, the rate was unaffected in 5 Hz, but appeared to slow after 30 Hz stimulation.

Figure 17 shows responses to spinal stimulation during deep anesthesia in humans. Patients were studied while spontaneous respiration was absent. Black dots represent individual experiments during stimulation (Intra-Stim), while the gray dots reflect individual experiments post-stimulation. At 5 Hz stimulation, no change in tidal volume or respiratory rate was observed at levels C2-C7. At 30 Hz stimulation, small changes in tidal volume were observed and more substantial increases in respiratory rate were observed. At 90 Hz, substantial increases in tidal volume were observed at C3/4 and increases in rate were observed at C3/4 and C6.

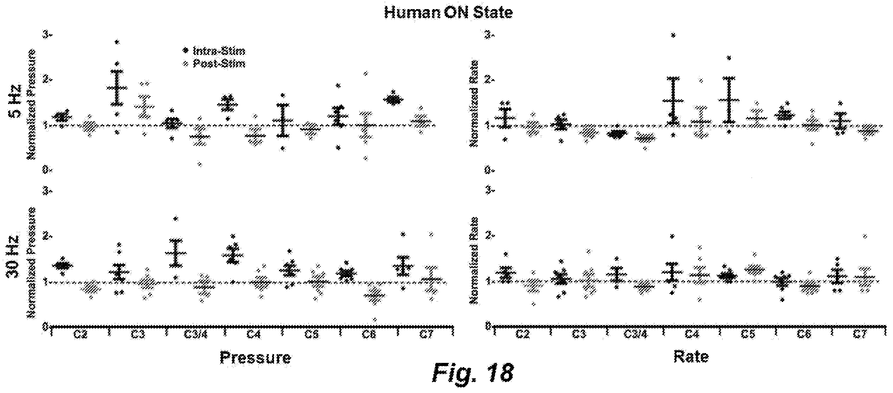

Figure 18 shows responses to spinal stimulation during light anesthesia in humans. Patients were studied while spontaneous respiration was present. Black dots represent individual experiments during stimulation (Intra-Stim), while the gray dots reflect individual experiments post-stimulation. At 5Hz stimulation, varying increases and decreases in tidal volume or respiratory rate were observed, normalized to pre-stimulation baseline. At 30 Hz stimulation, variable changes tidal volume and respiratory rate were observed. These results indicate that the cord may be more responsive to stimulation in the absence of anesthesia. Furthermore, it appears the tidal volume stimulation is short-lived.

### Example 4

### Thoracic and Lumbar Stimulation to Facilitate Respiration.

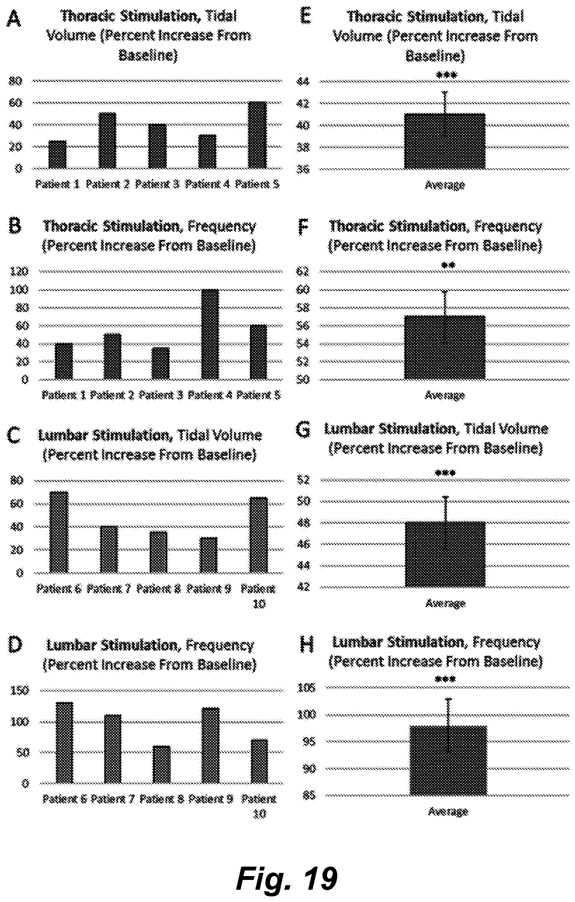

As illustrated in Figure 19, epidural stimulation (30 Hz) was performed in the thoracic (panels A, B, E, F) and lumbar (panels C, D, G, H) locations. Stimulation resulted in significant increases in both tidal volume (panels A, E, C, G) and frequency (panels B, F, D, H).

It is understood that the examples and embodiments described herein are for illustrative purposes only and that various modifications or changes in light thereof will be suggested to persons skilled in the art and are to be included within the purview of this application and scope of the appended claims.

## Claims

1. A monoaminergic agonist for use in a method of treatment of a subject with a respiratory deficiency to facilitate coughing, the opening of airways and/or swallowing in said subject, where said treatment further comprises:
neuromodulating the brain stem and/or suboccipital spinal cord, the cervical spinal cord, and/or the thoracic spinal cord, and/or the lumbar spinal cord, of said subject by administering transcutaneous stimulation to the brain stem and/or suboccipital spinal cord, the cervical spinal cord or a region thereof, and/or the thoracic spinal cord or a region thereof, and/or the lumbar spinal cord or a region thereof at a frequency and intensity sufficient to facilitate coughing, and/or the opening of airways and/or swallowing; and/or
neuromodulating the brain stem and/or suboccipital spinal cord, the cervical spinal cord or a region thereof, and/or the thoracic spinal cord or a region thereof, and/or the lumbar spinal cord or a region thereof, of said subject with a magnetic stimulator at a frequency and intensity sufficient to facilitate coughing, and/or the opening of airways and/or swallowing.

2. The monoaminergic agonist for use according to claim 1, wherein:
said treatment induces or facilitates coughing and/or clearing of airways in said subject, and said neuromodulating is at neuromodulating the brain stem and/or suboccipital spinal cord, the cervical spinal cord, and/or the thoracic spinal cord, and/or the lumbar spinal cord, of said subject.

3. The monoaminergic agonist for use according to claim 1, wherein said treatment induces or facilitates swallowing in said subject, and said neuromodulating is at neuromodulating the brain stem and/or suboccipital spinal cord, the cervical spinal cord, and/or the thoracic spinal cord, and/or the lumbar spinal cord, of said subject.

4. The monoaminergic agonist for use according to any one of claims 1-3, wherein said treatment comprises administering transcutaneous electrical stimulation wherein:
said transcutaneous stimulation is at a frequency of at least about 1 Hz, or at least about 2 Hz, or at least about 3 Hz, or at least about 4 Hz, or at least about 5 Hz, or at least about 10 Hz, or at least about 20 Hz or at least about 30 Hz or at least about 40 Hz or at least about 50 Hz or at least about 60 Hz or at least about 70 Hz or at least about 80 Hz or at least about 90 Hz or at least about 100 Hz, or at least about 200 Hz, or at least about 300 Hz, or at least about 400 Hz, or at least about 500 Hz, or at least about 1 kHz, or at least about 1.5 kHz, or at least about 2 kHz, or at least about 2.5 kHz, or at least about 5 kHz, or at least about 10 kHz, or up to about 25 kHz, or up to about 50 kHz, or up to about 100 kHz; and/or
said transcutaneous stimulation is at a frequency ranging from about 1 Hz, or from about 2 Hz, or from about 3 Hz, or from about 4 Hz, or from about 5 Hz, or from about 10 Hz, or from about 10 Hz, or from about 10 Hz, up to about 500 Hz, or up to about 400 Hz, or up to about 300 Hz, or up to about 200 Hz up to about 100 Hz, or up to about 90 Hz, or up to about 80 Hz, or up to about 60 Hz, or up to about 40 Hz, or from about 3 Hz or from about 5 Hz up to about 80 Hz, or from about 5 Hz to about 60 Hz, or up to about 30 Hz; or
said transcutaneous stimulation is at a frequency ranging from about 20 Hz or about 30 Hz to about 90 Hz or to about 100 Hz, to initiate respiration when no respiration pattern is present; and/or
said transcutaneous stimulation is at a frequency ranging from about 5 Hz or about 10 Hz up to about 90 Hz or about 100 Hz, when a respiration pattern is present; and/or
said transcutaneous stimulation is at an intensity ranging from about 5 mA or about 10 mA up to about 500 mA, or from about 5 mA or about 10 mA up to about 400 mA, or from about 5 mA or about 10 mA up to about 300 mA, or from about 5 mA or about 10 mA up to about 200 mA, or from about 5 mA or about 10 mA to up about 150 mA, or from about 5 mA or about 10 mA up to about 50 mA, or from about 5 mA or about 10 mA up to about 100 mA, or from about 5 mA or about 10 mA up to about 80 mA, or from about 5 mA or about 10 mA up to about 60 mA, or from about 5 mA or about 10 mA up to about 50 mA; and/or
said transcutaneous stimulation comprises administering pulses having a width that ranges from about 100 µs up to about 1 ms or up to about 800 µs, or up to about 600 µs, or up to about 500 µs, or up to about 400 µs, or up to about 300 µs, or up to about 200 µs, or up to about 100 µs, or from about 150 µs up to about 600 µs, or from about 200 µs up to about 500 µs, or from about 200 µs up to about 400 µs; and/or
said transcutaneous stimulation is at a frequency, pulse width, and amplitude sufficient to restore a resting respiration rate and at least 60%, or at least 70%, or at least 80%, or at least 90% of the subject's normal tidal volume; and/or
said transcutaneous stimulation is superimposed on a high frequency carrier signal; and/or
said high frequency carrier signal ranges from about 3 kHz, or about 5 kHz, or about 8 kHz up to about 30 kHz, or up to about 20 kHz, or up to about 15 kHz; and/or
said high frequency carrier signal is about 10 kHz; and/or
wherein said carrier frequency amplitude ranges from about 30 mA, or about 40 mA, or about 50 mA, or about 60 mA, or about 70 mA, or about 80 mA up to about 300 mA, or up to about 200 mA, or up to about 150 mA.

5. The monoaminergic agonist for use according to any one of claims 1-4, wherein said transcutaneous electrical stimulation is applied using a needle electrode.

6. The monoaminergic agonist for use according to any one of claims 1-3, wherein said treatment comprises administering magnetic neural stimulation wherein:
said magnetic stimulation produces a magnetic field of at least 1 tesla, or at least 2 tesla, or at least 3 tesla, or at least 4 tesla; and/or
said magnetic stimulation is at a frequency of at least about 1 Hz, or at least about 2 Hz, or at least about 3 Hz, or at least about 4 Hz, or at least about 5 Hz, or at least about 10 Hz, or at least about 20 Hz or at least about 30 Hz or at least about 40 Hz or at least about 50 Hz or at least about 60 Hz or at least about 70 Hz or at least about 80 Hz or at least about 90 Hz or at least about 100 Hz, or at least about 200 Hz, or at least about 300 Hz, or at least about 400 Hz, or at least about 500 Hz; and/or
said magnetic stimulation is at a frequency ranging from about 1 Hz, or from about 2 Hz, or from about 3 Hz, or from about 4 Hz, or from about 5 Hz, or from about 10 Hz, or from about 10 Hz, or from about 10 Hz, up to about 500 Hz, or up to about 400 Hz, or up to about 300 Hz, or up to about 200 Hz up to about 100 Hz, or up to about 90 Hz, or up to about 80 Hz, or up to about 60 Hz, or up to about 40 Hz, or from about 3 Hz or from about 5 Hz up to about 80 Hz, or from about 5 Hz to about 60 Hz, or up to about 30 Hz; and/or
said magnetic stimulation is at a frequency ranging from about 20 Hz or about 30 Hz to about 90 Hz or to about 100 Hz, to initiate respiration when no respiration pattern is present; and/or
said magnetic stimulation is at a frequency ranging from about 5 Hz or about 10 Hz up to about 90 Hz or about 100 Hz, when a respiration pattern is present; and/or
said magnetic stimulation is at a frequency, amplitude, and orientation sufficient to restore a resting respiration rate and at least 60%, or at least 70%, or at least 80%, or at least 90% of the subjects normal tidal volume.

7. The monoaminergic agonist for use according to any one of claims 1-6, wherein:
said transcutaneous electrical stimulation or said magnetic stimulation is applied over one or more regions selected from the group consisting of C0-C1, C0-C2, C0-C3, C0-C4, C0-C5, C0-C6, C0-C7, C0-T1, C1-C1, C1-C2, C1-C3, C1-C4, C1-C7, C1-C6, C1-C7, C1-T1, C2-C2, C2-C3, C2-C4, C2-C5, C2-C6, C2-C7, C2-T1, C3-C3, C3-C4, C3-C5, C3-C6, C3-C7, C3-T1, C4-C4, C4-C5, C4-C6, C4-C7, C4-T1, C5-C5, C5-C6, C5-C7, C5-T1, C6-C6, C6-C7, C6-T1, C7-C7, and C7-T1; and/or
said transcutaneous electrical stimulation or said magnetic stimulation is applied over one or more regions selected from the group consisting of T1-T1, T1-T2, T1-T3, T1-T4, T1-T5, T1-T6, T1-T7, T1-T8, T1-T9, T1-T10, T1-T11, T1-T12, T2-T2, T2-T3, T2-T4, T2-T5, T2-T6, T2-T7, T2-T8, T2-T9, T2-T10, T2-T11, T2-T12, T3-T3, T3-T4, T3-T5, T3-T6, T3-T7, T3-T8, T3-T9, T3-T10, T3-T11, T3-T12, T4-T4, T4-T5, T4-T6, T4-T7, T4-T8, T4-T9, T4-T10, T4-T11, T4-T12, T5-T5, T5-T6, T5-T7, T5-T8, T5-T9, T5-T10, T5-T11, T5-T12, T6-T6, T6-T7, T6-T8, T6-T9, T6-T10, T6-T11, T6-T12, T7-T7, T7-T8, T7-T9, T7-T10, T7-T11, T7-T12, T8-T8, T8-T9, T8-T10, T8-T11, T8-T12, T9-T9, T9-T10, T9-T11, T9-T12, T10-T10, T10-T11, T10-T12, T11-T11, T11-T12, and T12-T12; and/or
said transcutaneous electrical stimulation or said magnetic stimulation is applied over one or more regions from the group consisting of L1-L1, L1-L2 , L1-L3, L1-L4, L1-L5, L1-S1, L1-S2, L1-S3, L1-S4, L1-S5, L2-L2 , L2-L3, L2-L4, L2-L5, L2-S1, L2-S2, L2-S3, L2-S4, L2-S5, L3-L3, L3-L4, L3-L5, L3-S1, L3-S2, L3-S3, L3-S4, L3-S5, L4-L4, L4-L5, L4-S1, L4-S2, L4-S3, L4-S4, L4-S5, L5-L5 , L5-S1, L5-S2, L5-S3, L5-S4, L5-S5, S1-S1, S1-S2, S1-S3, S1-S4, S1-S5, S2-S2, S2-S3, S2-S4, S2-S5, S3-S3, S3-S4, S3-S5, S4-S4, S4-S5, and S5-S6; and/or
said transcutaneous electrical stimulation or said magnetic stimulation is applied over the coccyx.

8. The monoaminergic agonist for use according to any one of claims 1-7, wherein said monoaminergic agonist comprises an agent selected from the group consisting of a serotonergic drug, a dopaminergic drug, a noradrenergic drug, a GABAergic drug, and a glycinergic drug.

9. The monoaminergic agonist for use according to claim 8, wherein said agent is selected from the group consisting of buspirone, 8-hydroxy-2-(di-n-propylamino)tetralin (8-OH-DPAT), 4-(benzodioxan-5-yl)1-(indan-2-yl)piperazine (S15535), N-{2-[4-(2-methoxyphenyl)-1-piperazinyl]ethyl}-N- (2-pyridinyl)cyclo-hexanecarboxamide (WAY 100.635), Quipazine, Ketanserin, 4-amino-(6-chloro-2-pyridyl)-1 piperidine hydrochloride (SR 57227A), Ondanesetron, Methoxamine, Prazosin, Clonidine, Yohimbine, 6-chloro-1-phenyl-2,3,4,5-tetrahydro-1H-3-benzazepine-7,8-diol (SKF-81297), 7-chloro-3-methyl-1-phenyl-1,2,4,5-tetrahydro-3-benzazepin-8-ol (SCH-23390), Quinpirole, and Eticlopride.

## Patentansprüche

1. Monoaminerger Agonist zur Verwendung in einem Verfahren zur Behandlung eines Individuums mit einer Atemwegsdefizienz, um Husten, das Öffnen der Atemwege und/oder das Schlucken bei dem Individuum zu erleichtern, wobei die Behandlung weiters Folgendes umfasst:
das Neuromodulieren des Hirnstamms und/oder subokzipitalen Rückenmarks, des zervikalen Rückenmarks und/oder des thorakalen Rückenmarks und/oder des lumbalen Rückenmarks des Individuums durch Verabreichen transkutaner Stimulation an den Hirnstamm und/oder das subokzipitale Rückenmark, das zervikale Rückenmark oder eine Region davon und/oder das thorakale Rückenmark oder eine Region davon und/oder das lumbale Rückenmark oder eine Region davon mit einer Frequenz und Intensität, die ausreichen, um Husten und/oder das Öffnen der Atemwege und/oder das Schlucken zu erleichtern; und/oder
das Neuromodulieren des Hirnstamms und/oder subokzipitalen Rückenmarks, des zervikalen Rückenmarks oder einer Region davon und/oder des thorakalen Rückenmarks oder einer Region davon und/oder des lumbalen Rückenmarks oder einer Region davon des Individuums mit einem magnetischen Stimulator mit einer Frequenz und Intensität, die ausreichen, um Husten und/oder das Öffnen der Atemwege und/oder das Schlucken zu erleichtern.

2. Monoaminerger Agonist zur Verwendung nach Anspruch 1, wobei:
die Behandlung das Husten und/oder das Freimachen der Atemwege bei dem Individuum auslöst und wobei das Neuromodulieren ein Neuromodulieren des Hirnstamms und/oder des subokzipitalen Rückenmarks, des zervikalen Rückenmarks und/oder des thorakalen Rückenmars und/oder des lumbalen Rückenmarks des Individuums ist.

3. Monoaminerger Agonist zur Verwendung nach Anspruch 1, wobei die Behandlung das Schlucken bei dem Individuum auslöst oder erleichtert und wobei das Neuromodulieren ein Neuromodulieren des Hirnstamms und/oder des subokzipitalen Rückenmarks, des zervikalen Rückenmarks und/oder des thorakalen Rückenmars und/oder des lumbalen Rückenmarks des Individuums ist.

4. Monoaminerger Agonist zur Verwendung nach einem der Ansprüche 1 bis 3, wobei die Behandlung das Verabreichen einer transkutanen elektrischen Stimulation umfasst, wobei:
die transkutane Stimulation mit einer Frequenz von zumindest etwa 1 Hz oder zumindest etwa 2 Hz oder zumindest etwa 3 Hz oder zumindest etwa 4 Hz oder zumindest etwa 5 Hz oder zumindest etwa 10 Hz oder zumindest etwa 20 Hz oder zumindest etwa 30 Hz oder zumindest etwa 40 Hz oder zumindest etwa 50 Hz oder zumindest etwa 60 Hz oder zumindest etwa 70 Hz oder zumindest etwa 80 Hz oder zumindest etwa 90 Hz oder zumindest etwa 100 Hz oder zumindest etwa 200 Hz oder zumindest etwa 300 Hz oder zumindest etwa 400 Hz oder zumindest etwa 500 Hz oder zumindest etwa 1 kHz oder zumindest etwa 1,5 kHz oder zumindest etwa 2 kHz oder zumindest etwa 2,5 kHz oder zumindest etwa 5 kHz oder zumindest etwa 10 kHz oder bis zu etwa 25 kHz oder bis zu etwa 50 kHz oder bis zu etwa 100 kHz durchgeführt wird; und/oder
die transkutane Stimulation mit einer Frequenz im Bereich von etwa 1 Hz oder von etwa 2 Hz von etwa 3 Hz oder von etwa 4 Hz oder von etwa 5 Hz oder von etwa 10 Hz bis etwa 500 Hz oder bis zu etwa 400 Hz oder bis zu etwa 300 Hz oder bis zu etwa 200 Hz oder bis zu etwa 100 Hz oder bis zu etwa 90 Hz oder bis zu etwa 80 Hz oder bis zu etwa 60 Hz oder bis zu etwa 40 Hz oder von etwa 3 Hz oder von etwa 5 Hz bis etwa 80 Hz oder von etwa 5 Hz bis etwa 60 Hz oder bis zu etwa 30 Hz durchgeführt wird; oder
die transkutane Stimulation mit einer Frequenz im Bereich von etwa 20 Hz oder etwa 30 Hz bis etwa 90 Hz oder bis etwa 100 Hz durchgeführt wird, um die Atmung anzuregen, wenn kein Atemmuster vorhanden ist; und/oder
die transkutane Stimulation mit einer Frequenz im Bereich von etwa 5 Hz oder etwa 10 Hz bis zu etwa 90 Hz oder etwa 100 Hz durchgeführt wird, wenn ein Atemmuster vorhanden ist; und/oder
die transkutane Stimulation mit einer Intensität im Bereich von etwa 5 mA oder etwa 10 mA bis etwa 500 mA oder von etwa 5 mA oder etwa 10 mA bis etwa 400 mA oder von etwa 5 mA oder etwa 10 mA bis etwa 300 mA oder von etwa 5 mA oder etwa 10 mA bis etwa 200 mA oder von etwa 5 mA oder etwa 10 mA bis etwa 150 mA oder von etwa 5 mA oder etwa 10 mA bis etwa 50 mA oder von etwa 5 mA oder etwa 10 mA bis etwa 100 mA oder von etwa 5 mA oder etwa 10 mA bis etwa 80 mA oder von etwa 5 mA oder etwa 10 mA bis etwa 60 mA oder von etwa 5 mA oder etwa 10 mA bis etwa 50 mA durchgeführt wird; und/oder
die transkutane Stimulation die Verabreichung von Impulsen mit einer Breite, die im Bereich von etwa 100 µs bis etwa 1 ms oder bis etwa 800 µs oder bis etwa 600 µs oder bis etwa 500 µs oder bis etwa 400 µs oder bis etwa 300 µs oder bis etwa 200 µs oder bis etwa 100 µs oder von etwa 150 µs bis etwa 600 µs oder von etwa 200 µs bis etwa 500 µs oder von etwa 200 µs bis etwa 400 µs umfasst; und/oder
die transkutane Stimulation mit einer Frequenz, Impulsbreite und Amplitude durchgeführt wird, die ausreichen, um die Atemfrequenz im Ruhezustand auf zumindest 60 % oder zumindest 70 % oder zumindest 80 % oder zumindest 90 % des normalen Atemvolumens wiederherzustellen; und/oder
die transkutane Stimulation über ein Hochfrequenz-Trägersignal überlagert wird; und/oder
das Hochfrequenz-Trägersignal im Bereich von etwa 3 kHz oder etwa 5 kHz oder etwa 8 kHz bis zu etwa 30 kHz oder bis zu etwa 20 kHz oder bis zu 15 kHz liegt; und/oder
das Hochfrequenz-Trägersignal etwa 10 kHz beträgt; und/oder
wobei die Trägerfrequenz-Amplitude im Bereich von etwa 30 mA oder etwa 40 mA oder etwa 50 mA oder etwa 60 mA oder etwa 70 mA oder etwa 80 mA bis zu etwa 300 mA oder bis zu etwa 200 mA oder bis zu etwa 150 mA liegt.

5. Monoaminerger Agonist zur Verwendung nach einem der Ansprüche 1 bis 4, wobei die transkutane elektrische Stimulation unter Verwendung einer Nadelelektrode angewandt wird.

6. Monoaminerger Agonist zur Verwendung nach einem der Ansprüche 1 bis 3, wobei die Behandlung die Verabreichung magnetischer neuronaler Stimulation umfasst, wobei:
die magnetische Stimulation ein Magnetfeld mit zumindest 1 Tesla oder zumindest 2 Tesla oder zumindest 3 Tesla oder zumindest 4 Tesla erzeugt; und/oder
die magnetische Stimulation mit einer Frequenz von zumindest etwa 1 Hz oder zumindest etwa 2 Hz oder zumindest etwa 3 Hz oder zumindest etwa 4 Hz oder zumindest etwa 5 Hz oder zumindest etwa 10 Hz oder zumindest etwa 20 Hz oder zumindest etwa 30 Hz oder zumindest etwa 40 Hz oder zumindest etwa 50 Hz oder zumindest etwa 60 Hz oder zumindest etwa 70 Hz oder zumindest etwa 80 Hz oder zumindest etwa 90 Hz oder zumindest etwa 100 Hz oder zumindest etwa 200 Hz oder zumindest etwa 300 Hz oder zumindest etwa 400 Hz oder zumindest etwa 500 Hz durchgeführt wird; und/oder
die magnetische Stimulation mit einer Frequenz im Bereich von etwa 1 Hz oder von etwa 2 Hz von etwa 3 Hz oder von etwa 4 Hz oder von etwa 5 Hz oder von etwa 10 Hz bis etwa 500 Hz oder bis zu etwa 400 Hz oder bis zu etwa 300 Hz oder bis zu etwa 200 Hz oder bis zu etwa 100 Hz oder bis zu etwa 90 Hz oder bis zu etwa 80 Hz oder bis zu etwa 60 Hz oder bis zu etwa 40 Hz oder von etwa 3 Hz oder von etwa 5 Hz bis etwa 80 Hz oder von etwa 5 Hz bis etwa 60 Hz oder bis zu etwa 30 Hz durchgeführt wird; und/oder
die magnetische Stimulation mit einer Frequenz im Bereich von etwa 20 Hz oder etwa 30 Hz bis etwa 90 Hz oder bis etwa 100 Hz durchgeführt wird, um die Atmung anzuregen, wenn kein Atemmuster vorhanden ist; und/oder
die magnetische Stimulation mit einer Frequenz im Bereich von etwa 5 Hz oder etwa 10 Hz bis zu etwa 90 Hz oder etwa 100 Hz durchgeführt wird, wenn ein Atemmuster vorhanden ist; und/oder
die magnetische Stimulation mit einer Frequenz, Amplitude und Ausrichtung durchgeführt wird, die ausreichen, um die Atemfrequenz im Ruhezustand auf zumindest 60 % oder zumindest 70 % oder zumindest 80 % oder zumindest 90 % des normalen Atemvolumens wiederherzustellen.

7. Monoaminerger Agonist zur Verwendung nach einem der Ansprüche 1 bis 6, wobei:
die transkutane elektrische Stimulation oder die magnetische Stimulation über eine oder mehrere Regionen angewandt wird, die aus der aus den folgenden bestehenden Gruppe ausgewählt sind: C0-C1, C0-C2, C0-C3, C0-C4, C0-C5, C0-C6, C0-C7, C0-T1, C1-C1, C1-C2, C1-C3, C1-C4, C1-C7, C1-C6, C1-C7, C1-T1, C2-C2, C2-C3, C2-C4, C2-C5, C2-C6, C2-C7, C2-T1, C3-C3, C3-C4, C3-C5, C3-C6, C3-C7, C3-T1, C4-C4, C4-C5, C4-C6, C4-C7, C4-T1, C5-C5, C5-C6, C5-C7, C5-T1, C6-C6, C6-C7, C6-T1, C7-C7 und C7-T1; und/oder
die transkutane elektrische Stimulation oder die magnetische Stimulation über eine oder mehrere Regionen angewandt wird, die aus der aus den folgenden bestehenden Gruppe ausgewählt sind: T1-T1, T1-T2, T1-T3, T1-T4, T1-T5, T1-T6, T1-T7, T1-T8, T1-T9, T1-T10, T1-T11, T1-T12, T2-T2, T2-T3, T2-T4, T2-T5, T2-T6, T2-T7, T2-T8, T2-T9, T2-T10, T2-T11, T2-T12, T3-T3, T3-T4, T3-T5, T3-T6, T3-T7, T3-T8, T3-T9, T3-T10, T3-T11, T3-T12, T4-T4, T4-T5, T4-T6, T4-T7, T4-T8, T4-T9, T4-T10, T4-T11, T4-T12, T5-T5, T5-T6, T5-T7, T5-T8, T5-T9, T5-T10, T5-T11, T5-T12, T6-T6, T6-T7, T6-T8, T6-T9, T6-T10, T6-T11, T6-T12, T7-T7, T7-T8, T7-T9, T7-T10, T7-T11, T7-T12, T8-T8, T8-T9, T8-T10, T8-T11, T8-T12, T9-T9, T9-T10, T9-T11, T9-T12, T10-T10, T10-T11, T10-T12, T11-T11, T11-T12 und T12-T12; und/oder
die transkutane elektrische Stimulation oder die magnetische Stimulation über eine oder mehrere Regionen angewandt wird, die aus der aus den folgenden bestehenden Gruppe ausgewählt sind: L1-L1, L1-L2 , L1-L3, L1-L4, L1-L5, L1-S1, L1-S2, L1-S3, L1-S4, L1-S5, L2-L2 , L2-L3, L2-L4, L2-L5, L2-S1, L2-S2, L2-S3, L2-S4, L2-S5, L3-L3, L3-L4, L3-L5, L3-S1, L3-S2, L3-S3, L3-S4, L3-S5, L4-L4, L4-L5, L4-S1, L4-S2, L4-S3, L4-S4, L4-S5, L5-L5, L5-S1, L5-S2, L5-S3, L5-S4, L5-S5, S1-S1, S1-S2, S1-S3, S1-S4, S1-S5, S2-S2, S2-S3, S2-S4, S2-S5, S3-S3, S3-S4, S3-S5, S4-S4, S4-S5 und S5-S6; und/oder
die transkutane elektrische Stimulation oder die magnetische Stimulation über das Steißbein angewandt wird.

8. Monoaminerger Agonist zur Verwendung nach einem der Ansprüche 1 bis 7, wobei der monoaminerger Agonist ein Mittel umfasst, das aus der aus einem serotonergen Arzneimittel, einem dopaminergen Arzneimittel, einem noradrenergen Arzneimittel, einem GABAnergen Arzneimittel und einem glycerinergen Arzneimittel bestehenden Gruppe ausgewählt ist.

9. Monoaminerger Agonist zur Verwendung nach Anspruch 8, wobei das Mittel aus der aus den folgenden bestehenden Gruppe ausgewählt ist: Buspiron, 8-Hydroxy-2-(di-n-propyl-amino)tetralin (8-OH-DPAT), 4-(Benzodioxan-5-yl)-1-(indan-2-yl)piperazin (S15535), N-{2-[4-(2-Methoxyphenyl)-1-piperazinyl]ethyl}-N-(2-pyridinyl)cyclohexancarboxamid (WAY 100.635), Quipazin, Ketanserin, 4-Amino-(6-chlor-2-pyridyl)-1-piperidin-hydrochlorid (SR 57227A), Ondanesetron, Methoxamin, Prazosin, Clonidin, Yohimbin, 6-Chlor-1-phenyl-2,3,4,5-tetrahydro-1H-3-benzazepin-7,8-diol (SKF-81297), 7-Chlor-3-methyl-1-phenyl-1,2,4,5-tetrahydro-3-benzazepin-8-ol (SCH-23390), Quinpirol und Eticloprid.

## Revendications

1. Agoniste monoaminergique pour une utilisation dans une méthode de traitement d'un sujet souffrant d'une insuffisance respiratoire pour faciliter le fait de tousser, l'ouverture des voies respiratoires et/ou la déglutition chez ledit sujet, ledit traitement comprenant en outre :
la neuromodulation du tronc cérébral et/ou de la moelle épinière sous-occipitale, de la moelle épinière cervicale et/ou de la moelle épinière thoracique, et/ou de la moelle épinière lombaire, dudit sujet par administration d'une stimulation transcutanée au tronc cérébral et/ou à la moelle épinière sous-occipitale, la moelle épinière cervicale ou une région de celle-ci, et/ou la moelle épinière thoracique ou une région de celle-ci, et/ou de la moelle épinière lombaire ou une région de celle-ci à une fréquence et une intensité suffisantes pour faciliter le fait de tousser, et/ou l'ouverture des voies respiratoires et/ou la déglutition ; et/ou
la neuromodulation du tronc cérébral et/ou de la moelle épinière sous-occipitale, de la moelle épinière cervicale ou d'une région de celle-ci, et/ou de la moelle épinière thoracique ou d'une région de celle-ci, et/ou de la moelle épinière lombaire ou d'une région de celle-ci, dudit sujet avec un stimulateur magnétique à une fréquence et une intensité suffisantes pour faciliter le fait de tousser, et/ou l'ouverture des voies respiratoires et/ou la déglutition.

2. Agoniste monoaminergique pour une utilisation selon la revendication 1, dans lequel :
ledit traitement induit ou facilite le fait de tousser et/ou le dégagement des voies respiratoires chez ledit sujet, et ladite neuromodulation vise à neuromoduler le tronc cérébral et/ou la moelle épinière sous-occipitale, la moelle épinière cervicale, et/ou la moelle épinière thoracique, et/ou la moelle épinière lombaire, dudit sujet.

3. Agoniste monoaminergique pour une utilisation selon la revendication 1, dans lequel ledit traitement induit ou facilite la déglutition chez ledit sujet, et ladite neuromodulation vise à neuromoduler le tronc cérébral et/ou la moelle épinière sous-occipitale, la moelle épinière cervicale, et/ou la moelle épinière thoracique, et/ou la moelle épinière lombaire, dudit sujet.

4. Agoniste monoaminergique selon l'une quelconque des revendications 1 à 3, dans lequel ledit traitement comprend l'administration d'une stimulation électrique transcutanée dans lequel :
ladite stimulation transcutanée est effectuée à une fréquence d'au moins environ 1 Hz, ou d'au moins environ 2 Hz, ou d'au moins environ 3 Hz, ou d'au moins environ 4 Hz, ou d'au moins environ 5 Hz, ou d'au moins environ 10 Hz, ou d'au moins environ 20 Hz ou d'au moins environ 30 Hz ou d'au moins environ 40 Hz ou d'au moins environ 50 Hz ou d'au moins environ 60 Hz ou d'au moins environ 70 Hz ou d'au moins environ 80 Hz ou d'au moins environ 90 Hz ou d'au moins environ 100 Hz, ou d'au moins environ 200 Hz, ou d'au moins environ 300 Hz, ou d'au moins environ 400 Hz, ou d'au moins environ 500 Hz, ou d'au moins environ 1 kHz, ou d'au moins environ 1,5 kHz, ou d'au moins environ 2 kHz, ou d'au moins environ 2,5 kHz, ou au moins environ 5 kHz, ou au moins environ 10 kHz, ou jusqu'à environ 25 kHz ou jusqu'à environ 50 kHz, ou jusqu'à environ 100 kHz ; et/ou
ladite stimulation transcutanée est effectuée à une fréquence comprise dans la plage allant d'environ 1 Hz, ou d'environ 2 Hz, ou d'environ 3 Hz, ou d'environ 4 Hz, ou d'environ 5 Hz, ou d'environ 10 Hz, ou d'environ 10 Hz, ou d'environ 10 Hz, jusqu'à environ 500 Hz, ou jusqu'à environ 400 Hz, ou jusqu'à environ 300 Hz, ou jusqu'à environ 200 Hz jusqu'à environ 100 Hz, ou jusqu'à environ 90 Hz, ou jusqu'à environ 80 Hz, ou jusqu'à environ 60 Hz, ou jusqu'à environ 40 Hz, ou d'environ 3 Hz ou d'environ 5 Hz jusqu'à environ 80 Hz, ou d'environ 5 Hz à environ 60 Hz, ou jusqu'à environ 30 Hz ; ou
ladite stimulation transcutanée est effectuée à une fréquence comprise dans la plage allant d'environ 20 Hz ou d'environ 30 Hz à environ 90 Hz ou à environ 100 Hz, pour initier la respiration lorsqu'un schéma respiratoire n'est pas présent ; et/ou
ladite stimulation transcutanée est effectuée à une fréquence comprise dans la plage allant d'environ 5 Hz ou d'environ 10 Hz jusqu'à environ 90 Hz ou environ 100 Hz, lorsqu'un schéma respiratoire est présent ; et/ou
ladite stimulation transcutanée est effectuée à une fréquence comprise dans la plage allant d'environ 5 mA ou d'environ 10 mA jusqu'à environ 500 mA, ou d'environ 5 mA ou d'environ 10 mA jusqu'à environ 400 mA, ou d'environ 5 mA ou d'environ 10 mA jusqu'à environ 300 mA, ou d'environ 5 mA ou d'environ 10 mA jusqu'à environ 200 mA, ou d'environ 5 mA ou d'environ 10 mA jusqu'à environ 150 mA, ou d'environ 5 mA ou d'environ 10 mA jusqu'à environ 50 mA, ou d'environ 5 mA ou d'environ 10 mA jusqu'à environ 100 mA, ou d'environ 5 mA ou d'environ 10 mA jusqu'à environ 80 mA, ou d'environ 5 mA ou d'environ 10 mA jusqu'à environ 60 mA, ou d'environ 5 mA ou d'environ 10 mA jusqu'à environ 50 mA ; et/ou
ladite stimulation transcutanée comprend l'administration d'impulsions ayant une largeur qui est comprise dans la plage allant d'environ 100 µs jusqu'à environ 1 ms ou jusqu'à environ 800 µs, ou jusqu'à environ 600 µs, ou jusqu'à environ 500 µs, ou jusqu'à environ 400 µs, ou jusqu'à environ 300 µs, ou jusqu'à environ 200 µs, ou jusqu'à environ 100 µs, ou d'environ 150 µs jusqu'à environ 600 µs, ou d'environ 200 µs jusqu'à environ 500 µs, ou d'environ 200 µs jusqu'à environ 400 µs ; et/ou
ladite stimulation transcutanée est effectuée à une fréquence, une largeur d'impulsion et une amplitude suffisantes pour rétablir une fréquence respiratoire au repos et au moins 60 %, ou au moins 70 %, ou au moins 80 %, ou au moins 90 % du volume courant normal du sujet ; et/ou
ladite stimulation transcutanée est superposée à un signal porteur haute fréquence ; et/ou
ledit signal porteur haute fréquence est compris dans la plage allant d'environ 3 kHz, ou d'environ 5 kHz, ou d'environ 8 kHz jusqu'à environ 30 kHz, ou jusqu'à environ 20 kHz ou jusqu'à environ 15 kHz ; et/ou
ledit signal porteur haute fréquence est d'environ 10 kHz ; et/ou
dans lequel ladite amplitude de la fréquence porteuse est comprise dans la plage allant d'environ 30 mA, ou d'environ 40 mA, ou d'environ 50 mA, ou d'environ 60 mA, ou d'environ 70 mA, ou d'environ 80 mA jusqu'à environ 300 mA, ou jusqu'à environ 200 mA, ou jusqu'à environ 150 mA.

5. Agoniste monoaminergique pour une utilisation selon l'une quelconque des revendications 1 à 4, dans lequel ladite stimulation électrique transcutanée est appliquée en utilisant une électrode-aiguille.

6. Agoniste monoaminergique pour une utilisation selon l'une quelconque des revendications 1 à 3, dans lequel ledit traitement comprend l'administration d'une stimulation neuronale magnétique, dans lequel :
ladite stimulation magnétique produit un champ magnétique d'au moins 1 tesla, ou d'au moins 2 teslas, ou d'au moins 3 teslas, ou d'au moins 4 teslas ; et/ou
ladite stimulation magnétique est effectuée à une fréquence d'au moins environ 1 Hz, ou d'au moins environ 2 Hz, ou d'au moins environ 3 Hz, ou d'au moins environ 4 Hz, ou d'au moins environ 5 Hz, ou d'au moins environ 10 Hz, ou d'au moins environ 20 Hz, ou d'au moins environ 30 Hz ou d'au moins environ 40 Hz ou d'au moins environ 50 Hz ou d'au moins environ 60 Hz ou d'au moins environ 70 Hz ou d'au moins environ 80 Hz ou d'au moins environ 90 Hz ou d'au moins environ 100 Hz, ou d'au moins environ 200 Hz, ou d'au moins environ 300 Hz, ou d'au moins environ 400 Hz, ou d'au moins environ 500 Hz ; et/ou
ladite stimulation magnétique est effectuée à une fréquence comprise dans la plage allant d'environ 1 Hz, ou d'environ 2 Hz, ou d'environ 3 Hz, ou d'environ 4 Hz, ou d'environ 5 Hz, ou d'environ 10 Hz, ou d'environ 10 Hz, jusqu'à environ 500 Hz, ou jusqu'à environ 400 Hz, ou jusqu'à environ 300 Hz, ou jusqu'à environ 200 Hz, jusqu'à environ 100 Hz, ou jusqu'à environ 90 Hz, ou jusqu'à environ 80 Hz, ou jusqu'à environ 60 Hz, ou jusqu'à environ 40 Hz, ou d'environ 3 Hz ou d'environ 5 Hz jusqu'à environ 80 Hz, ou d'environ 5 Hz à environ 60 Hz, ou jusqu'à environ 30 Hz ; et/ou
ladite stimulation magnétique est effectuée à une fréquence comprise dans la plage allant d'environ 20 Hz ou d'environ 30 Hz à environ 90 Hz ou à environ 100 Hz, pour initier la respiration lorsqu'un schéma respiratoire n'est pas présent ; et/ou
ladite stimulation transcutanée est effectuée à une fréquence comprise dans la plage d'environ 5 Hz ou d'environ 10 Hz à environ 90 Hz ou environ 100 Hz, lorsqu'un schéma respiratoire est présent ; et/ou
ladite stimulation magnétique est effectuée à une fréquence, une amplitude et une orientation suffisantes pour rétablir une fréquence respiratoire de repos et au moins 60 %, ou au moins 70 %, ou au moins 80 %, ou au moins 90 % du volume courant normal du sujet.

7. Agoniste monoaminergique pour une utilisation selon l'une quelconque des revendications 1 à 6, dans lequel :
ladite stimulation électrique transcutanée ou ladite stimulation magnétique est appliquée sur une ou plusieurs régions choisies dans le groupe constitué par C0-C1, C0-C2, C0-C3, C0-C4, C0-C5, C0-C6, C0-C7, C0-T1, C1-C1, C1-C2, C1-C3, C1-C4, C1-C7, C1-C6, C1-C7, C1-T1, C2-C2, C2-C3, C2-C4, C2-C5, C2-C6, C2-C7, C2-T1, C3-C3, C3-C4, C3-C5, C3-C6, C3-C7, C3-T1, C4-C4, C4-C5. C4-C6, C4-C7, C4-T1, C5-C5, C5-C6, C5-C7, C5-T1, C6-C6, C6-C7, C6-T1, C7-C7 et C7-T1 ; et/ou
ladite stimulation électrique transcutanée ou ladite stimulation magnétique est appliquée sur une ou plusieurs régions sélectionnées dans le groupe constitué par T1-T1, T1-T2, T1-T3, T1-T4, T1-T5, T1-T6, T1-T7, T1-T8, T1-T9, T1-T10, T1-T11, T1-T12, T2-T2, T2-T3, T2-T4, T2-T5, T2-T6, T2-T7, T2-T8, T2-T9, T2-T10, T2-T11, T2-T12, T3-T3, T3-T4, T3-T5, T3-T6, T3-T7, T3-T8, T3-T9, T3-T10, T3-T11, T3-T12, T4-T4, T4-T5, T4-T6, T4-T7, T4-T8, T4-T9, T4-T10, T4-T11, T4-T12, T5-T5, T5-T6, T5-T7, T5-T8, T5-T9, T5-T10, T5-T11, T5-T12, T6-T6, T6-T7, T6-T8, T6-T9, T6-T10, T6-T11, T6-T12, T7-T7, T7-T8, T7-T9, T7-T10, T7-T11, T7-T12, T8-T8, T8-T9, T8-T10, T8-T11, T8-T12, T9-T9, T9-T10, T9-T11, T9-T12, T10-T10, T10-T11, T10-T12, T11-T11, T11-T12 et T12-T12 ; et/ou
ladite stimulation électrique transcutanée ou ladite stimulation magnétique est appliquée sur une ou plusieurs régions du groupe constitué par L1-L1, L1-L2, L1-L3, L1-L4, L1-L5, L1-S1, L1-S2, L1-S3, L1-S4, L1-S5, L2-L2, L2-L3, L2-L4, L2-L5, L2-S1, L2-S2, L2-S3, L2-S4, L2-S5, L3-L3, L3-L4, L3-L5, L3-S1, L3-S2, L3-S3, L3-S4, L3-S5, L4-L4, L4-L5, L4-S1, L4-S2, L4-S3, L4-S4, L4-S5, L5-L5, L5-S1, L5-S2, L5-S3, L5-S4, L5-S5, S1-S1, S1-S2, S1-S3, S1-S4, S1-S5, S2-S2, S2-S3, S2-S4, S2-S5, S3-S3, S3-S4, S3-S5, S4-S4, S4-S5 et S5-S6 ; et/ou
ladite stimulation électrique transcutanée ou ladite stimulation magnétique est appliquée sur le coccyx.

8. Agoniste monoaminergique pour une utilisation selon l'une quelconque des revendications 1 à 7, dans lequel ledit agoniste monoaminergique comprend un agent choisi dans le groupe constitué par un médicament sérotoninergique, un médicament dopaminergique, un médicament noradrénergique, un médicament GABAergique et un médicament glycinergique.

9. Agoniste monoaminergique pour une utilisation selon la revendication 8, dans lequel ledit agent est choisi dans le groupe constitué par la buspirone, la 8-hydroxy-2-(di-n-propylamino)tétraline (8-OH-DPAT), la 4-(benzodioxan-5-yl)-1-(indan-2-yl)pipérazine (S15535), le N-{2-[4-(2-méthoxyphényl)-1-pipérazinyl]éthyl}-N-(2-pyridinyl)cyclohexanecarboxamide (WAY 100.635), la quipazine, la kétansérine, le chlorhydrate de 4-amino-(6-chloro-2-pyridyl)-1-pipéridine (SR 57227A), l'ondansétron, la méthoxamine, la prazosine, la clonidine, l'yohimbine, le 6-chloro-1-phényl-2,3,4,5-tétrahydro-1H-3-benzazépine-7,8-diol (SKF-81297), le 7-chloro-3-méthyl-1-phényl-1,2,4,5-tétrahydro-3-benzazépin-8-ol (SCH-23390), le quinpirole et l'éticlopride.
